(19) Europäisches Patentamt / European Patent Office / Office européen des brevets

(11) **EP 0 787 796 B1**

(12) **FASCICULE DE BREVET EUROPEEN**

(45) Date de publication et mention de la délivrance du brevet:
**14.01.2009 Bulletin 2009/03**

(51) Int Cl.:
*C12N 9/88* (2006.01)    *C12N 15/60* (2006.01)
*A61K 39/10* (2006.01)    *C07K 16/40* (2006.01)
*C12P 21/08* (2006.01)    *A61K 39/395* (2006.01)
*G01N 33/577* (2006.01)    *G01N 33/569* (2006.01)
*A61K 31/70* (2006.01)

(21) Numéro de dépôt: 96401402.1

(22) Date de dépôt: **25.06.1996**

(54) **Epitopes protecteurs de l'adényl cyclase-hémolysine (AC-Hly). leur application au traitement ou à la prévention des infections par Bordetella**

Schützende Epitope von Adenylate-Zyklase-Hämolysin(AC-Hly) und ihre Verwendung zur Behandlung oder Verhütung von Bordetella-Infektionen

Protective epitopes from adenylate cyclase-hemolysin(AC-Hly) and their use for treatment or prevention of Bordetella infections

(84) Etats contractants désignés:
**AT BE CH DE DK ES FI FR GB GR IE IT LI LU MC NL PT SE**

(30) Priorité: **30.06.1995 FR 9507945**

(43) Date de publication de la demande:
**06.08.1997 Bulletin 1997/32**

(73) Titulaire: **INSTITUT PASTEUR 75724 Paris Cedex 15 (FR)**

(72) Inventeurs:
 • **Betsou, Fotini**
   **75015 Paris (FR)**
 • **Sebo, Peter**
   **CZ 140 00 Praque 4-Krc (CZ)**
 • **Nato, Farida**
   **92160 Antony (FR)**
 • **Landais, Stéphanie**
   **75015 Paris (FR)**
 • **Guiso, Nicole**
   **FR-75008 Paris (FR)**

(74) Mandataire: **Desaix, Anne et al
Ernest Gutmann - Yves Plasseraud S.A.S.
3, rue Auber
75009 Paris (FR)**

(56) Documents cités:
**FR-A- 2 728 170**

• **INFECTION AND IMMUNITY, vol. 61, no. 9, Septembre 1993, WASHINGTON US, pages 3583-3589, XP002016441 F. BETSOU ET AL.: "CyaC-MEDIATED ACTIVATION IS IMPORTANT NOT ONLY FOR TOXIC BUT ALSO FOR PROTECTIVE ACTIVITIES OF BORDETELLA PERTUSSIS ADENYLATE CYCLASE-HEMOLYSIN."**
• **SCIENCE, vol. 266, 21 Octobre 1994, LANCASTER, PA US, pages 433-435, XP002016442 M. HACKETT ET AL.: "INTERNAL LYSINE PALMITOYLATION IN ADENYLATE CYCLASE TOXIN FROM BORDETELLA PERTUSSIS."**
• **PROCEEDINGS OF THE NATIONAL ACADEMY OF SCIENCES OF USA, vol. 89, Juin 1992, WASHINGTON US, pages 4898-4902, XP002016443 M.K. GROSS ET AL.: "TARGETED MUTATIONS THAT ABLATE EITHER THE ADENYLATE CYCLASE OR HEMOLYSIN FUNCTION OF THE BIFUNCTIONAL cyaA TOXIN OF BORDETELLA PERTUSSIS ABOLISH VIRULENCE."**
• **JOURNAL OF BACTERIOLOGY, vol. 175, no. 2, Janvier 1993, BALTIMORE, MD, US, pages 519-527, XP000563855 D.T. BEATTIE ET AL.: "REPRESSOR BINDING TO A REGULATORY SITE IN THE DNA CODING SEQUENCE IS SUFFICIENT TO CONFER TRANSCRIPTIONAL REGULATION OF THE vir-REPRESSED GENES (vrg GENES) IN BORDETELLA PERTUSSIS."**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 0 787 796 B1

- **INFECTION AND IMMUNITY, vol. 63, no. 9, Septembre 1995, WASHINGTON US, pages 3309-3315, XP002016444 F. BETSOU ET AL.: "THE C-TERMINAL DOMAIN IS ESSENTIAL FOR PROTECTIVE ACTIVITY OF THE BORDETELLA PERTUSSIS ADENYLATE CYCLASE-HEMOLYSIN."**

**Description**

La présente demande concerne des séquences d'acides aminés comprenant des épitopes de l'adényl cyclase - hémolysine de Bordetella. L'adénylcyclase-hémolysine (AC-Hly) est une des toxines participant au syndrome infectieux de Bordetella. L'AC-Hly est une protéine bifonctionnelle, possédant une activité adényl cyclase et une activité hémolytique. Elle est sécrétée par la bactérie. Son gène de structure a été cloné et séquencé (Glaser P. et al, 1988, Molec. Microb. 2, 19-20). Il s'avère que cette protéine fait partie de la famille des toxines dites « RTX » pour « repeats in toxins » et présente des homologies avec l'hémolysine d'Escherichia coli, d'Actinobacillus pleuropneumoniae, les leucotoxines de Pasteurella haemolytica et d'Actinobacillus actinomycetemcomitans. Cette protéine, comme la PTX (toxine de pertussis), est capable de pénétrer dans les cellules eucaryotes telles que les macrophages, d'être activée par la calmoduline, de synthétiser de grandes quantités d'AMPc et de perturber les fonctions cellulaires (Coote J. 1992. FEMS Microbiol. Rev. 88:137-162).

Les inventeurs ont identifié au sein de cette séquence, différents domaines ayant la capacité d'induire la formation d'anticorps protecteurs contre une infection par Bordetella en particulier par B. pertussis, ou/et B. parapertussis et/ou B. bronchiseptica.

L'invention a donc pour objet des séquences d'acides aminés susceptibles d'entrer dans la constitution de compositions immunogènes ou de vaccins protecteurs contre des infections à Bordetella ainsi que des anticorps dirigés contre ces séquences d'acides aminés, utilisables par exemple en immunothérapie. L'immunothérapie ou sérothérapie est notamment applicable chez des enfants, le cas échéant chez des nourrissons infectés par B. pertussis, B. parapertussis ou B. bronchiseptica. L'invention propose des applications en médecine humaine ou en médecine vétérinaire.

La présente invention a pour objet une séquence d'acides aminés dérivée de la séquence polypeptidique de l'adenylcyclase-hémolysine (AC-Hly), caractérisée en ce qu'elle est capable d'induire la formation d'anticorps protecteurs contre une infection par B. pertussis et/ou B. parapertussis et/ou B. bronchiseptica et en ce qu'elle est choisie parmi les enchaînements suivants :

a) la séquence d'une protéine tronquée comprenant l'enchaînement des acides aminés situés approximativement entre la position 910, de préférence 913, et le dernier acide aminé C-terminal de la séquence polypeptidique de l'AC-Hly de *B. pertussis* ou d'une séquence correspondant à la précédente chez *B. parapertussis* ou chez *B. bronchiseptica,* la dite séquence comportant une modification par addition d'un acide gras entre les acides aminés 980 environ et 985 environ, de préférence au niveau de l'acide aminé 983 ;

b) une séquence ayant une conformation tridimensionnelle identique ou analogue à celle de la séquence polypeptidique correspondante de l'AC-Hly de *B. pertussis, B. parapertussis* ou *B. bronchiseptica,* comprenant un enchaînement d'acides aminés tel que défini ci-dessus en a) et un enchaînement de 16 à 500 acides aminés comprenant une séquence consistant en l'enchaînement des acides aminés 385 à 400, 385 à 450, ou 385 à 500 de la séquence polypeptidique de l'AC-Hly de *B. pertussis* ou d'une séquence correspondant à la précédente chez *B. parapertussis* ou chez *B. bronchiseptica* ces deux enchaînements étant réunis par l'intermédiaire d'une séquence antigénique dérivée d'une protéine différente de l'AC-Hly; et

c) une séquence ayant une conformation tridimensionnelle identique ou analogue à celle de la séquence polypeptidique correspondante de l'AC-Hly de *B. pertussis, B. parapertussis* ou *B. bronchiseptica,* comprenant un enchaînement d'acides aminés tel que défini ci-dessus en a) et un enchaînement de 16 à 500 acides aminés comprenant une séquence consistant en l'enchaînement des acides aminés 385 à 400, 385 à 450, ou 385 à 500 de la séquence polypeptidique de l'AC-Hly de *B. pertussis* ou d'une séquence correspondant à la précédente de *B. parapertussis* ou chez *B. bronchiseptica,* ces deux enchaînements. étant soit contigus, soit réunis par l'intermédiaire de l'enchaînement d'acides aminés naturellement présent entre les deux enchaînements définis ci-dessus au sein de l'AC-Hly de *B. pertussis, B. parapertussis* ou *B. bronchiseptica,* la séquence d'acides aminés obtenue étant tronquée par rapport à la séquence Ac-Hly de *Bordetella.*

La demande décrit également une séquence comprenant un enchaînement de 16 à 500 acides aminés comprenant les acides aminés 385 à 400 de la séquence polypeptidique de l'AC-Hly de *B. pertussis* ou d'une séquence correspondant à la précédente chez *B. parapertussis* ou chez *B. bronchiseptica,* capable d'induire la formation d'anticorps protecteurs contre une infection par *B. pertussis* et/ou *B. parapertussis* et/ou *B. bronchiseptica.*

Par l'expression « séquence d'acides aminés dérivée de la séquence polypeptidique de l'AC-Hly », on entend dans le cadre de l'invention, une séquence dont les acides aminés sont identiques par leur nature ou par leur enchaînement, à ceux de la séquence polypeptidique de l'AC-Hly ou sont pour certains d'entre eux substitués, délétés ou ajoutés, de façon telle que les propriétés immunologiques de l'AC-Hly sont conservées. En particulier, une telle séquence, dérivée de la séquence polypeptidique de l'AC-Hly est reconnue par des anticorps formés chez un patient infecté par Bordetella, notamment par B. pertussis, B. parapertussis ou B. bronchiseptica.

On considérera, dans le cadre de la présente invention, que la structure ou la conformation de l'AC-Hly est conservée, la séquence d'acides aminés de l'invention ayant alors une structure identique ou analogue à celle de l'AC-Hly, lorsque ladite séquence d'acides aminés est capable, après immunisation d'un patient ou d'un animal, d'induire une immunité protectrice contre les infections à Bordetella.

Une séquence selon l'invention peut être obtenue par protéolyse de l'AC-Hly purifiée de Bordetella. De préférence, cette séquence est obtenue par synthèse chimique ou par les techniques du génie génétique.

Ainsi, pour produire par génie génétique une séquence d'acides aminés selon l'invention, on aura recours à des plasmides portant des fragments du gène CyaA.

A titre d'exemple, la synthèse chimique peut être réalisée dans des automates de type Applied Biosystem. On peut aussi mettre en oeuvre la technique de Betsou F. et al, 1993, Infect. Immun., 61:3583-3589.

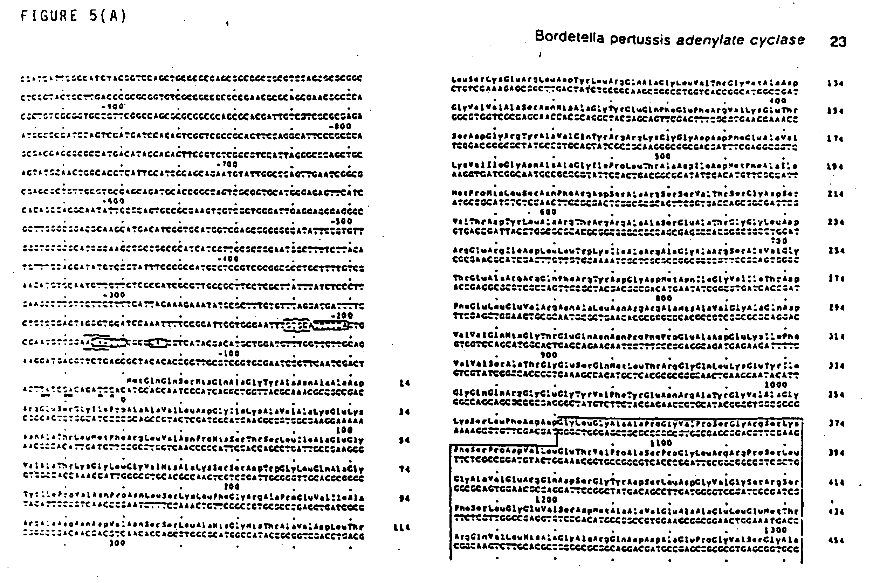

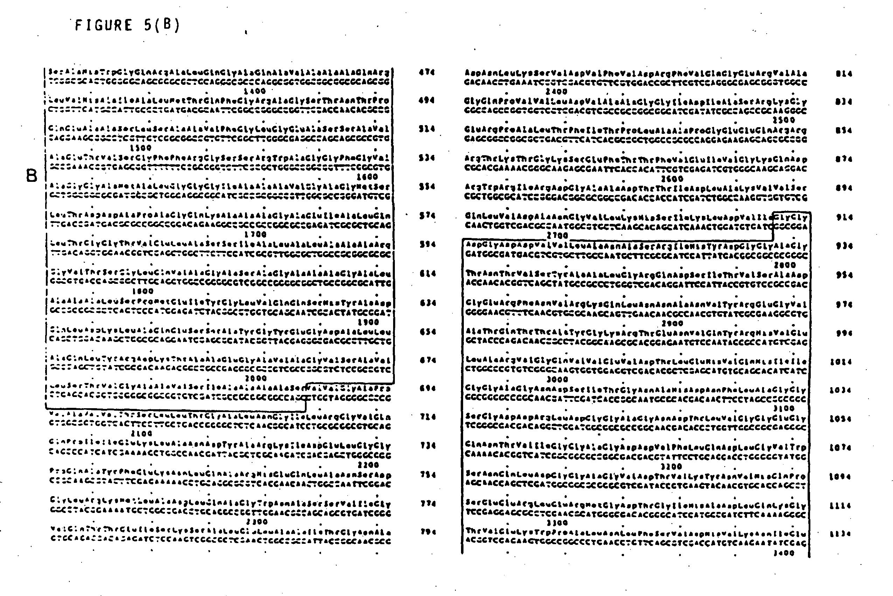

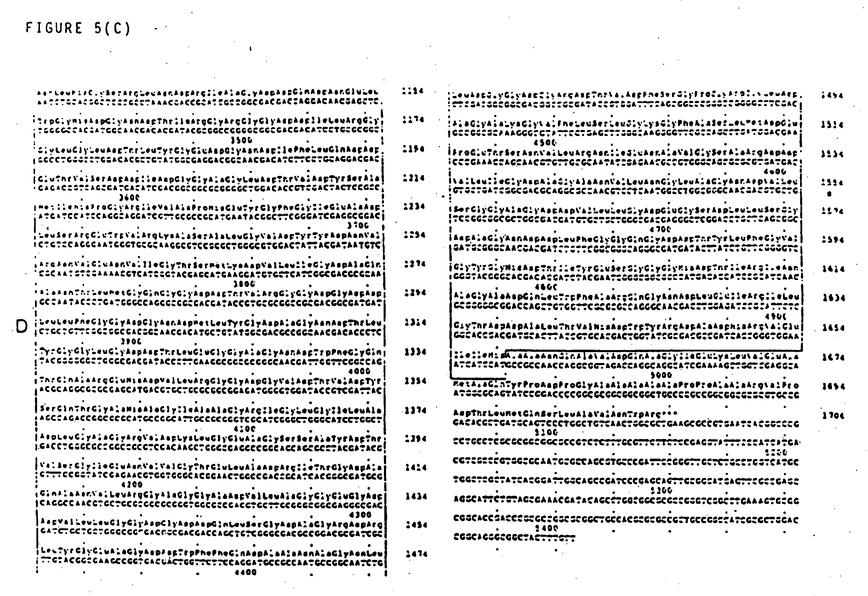

De cette façon, on pourra préparer un peptide correspondant à l'enchaînement des acides aminés 385 à 400, voire 385 à 500 de l'AC-Hly de B. pertussis ou à l'enchaînement correspondant de l'AC-Hly de B. parapertussis ou de B. bronchiseptica. La séquence d'acides aminés ainsi que la séquence nucléotidique de l'AC-Hly de B. pertussis a été décrite dans Glaser et al, 1988, Molec. Microb. 2-19-30 et est représentée à la figure 5. La séquence d'acides aminés et la séquence nucléotidique de l'AC-Hly de B. bronchiseptica est représentée à la figure b.

La séquence d'acides aminés de l'AC-Hly de B. parapertussis et la séquence nucléotidique codant pour l'AC-Hly peuvent être obtenues par les techniques classiques à partir de l'ADN d'une souche de B. parapertussis, par exemple la souche n° 1 déposée à la CNCM le 2 Décembre 1994 sous le n° I-1498.

Lorsqu'une séquence d'acides aminés selon l'invention est produite par génie génétique à partir de l'ADN des gènes CyaA de B. pertussis, B. parapertussis ou B. bronchiseptica, et lorsqu'elle comporte l'acide aminé en position 983 de l'AC-Hly de B. pertussis ou un acide aminé correspondant de l'AC-Hly de B. parapertussis ou B. bronchiseptica, on veillera à ce que cette séquence soit produite dans un hôte cellulaire exprimant également le gène CyaC des souches identifiées ci-dessus. L'expression du gène CyaC permet la modification par addition d'un acide gras nécessaire pour conserver à la séquence d'acides aminés comportant le résidu 983. L'addition d'un acide gras peut, à titre d'exemple, être une palmitoylation.

Des souches utilisables pour avoir accès aux gènes CyaA et CyaC sont par exemple B. pertussis HAV déposée à la CNCM le 19 Octobre 1994 sous le n° I-1485, B. parapertussis I-1498 et B. bronchiseptica 9.735 déposée à la CNCM le 12 Mai 1989 sous le n° 1-858.

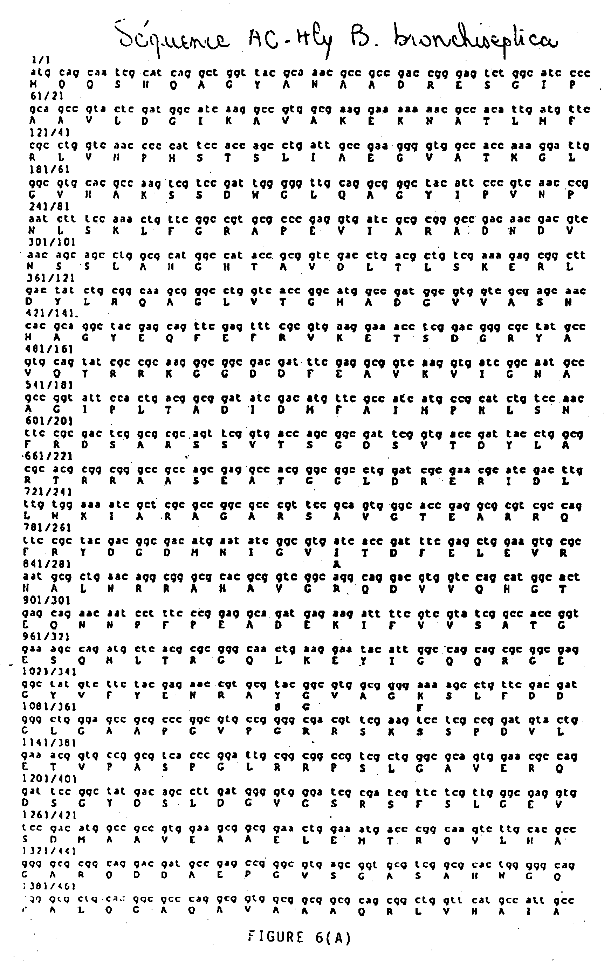

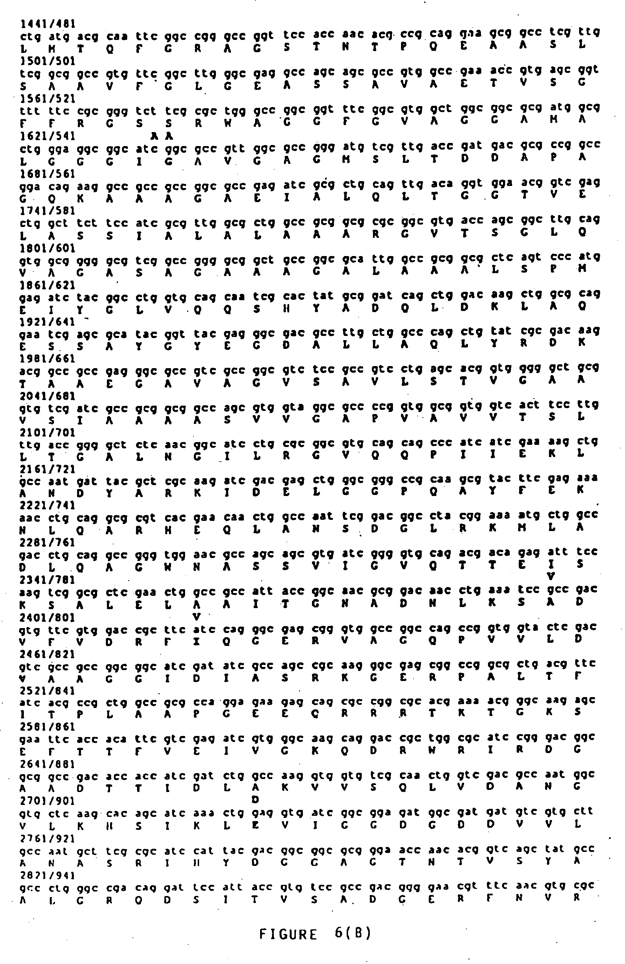

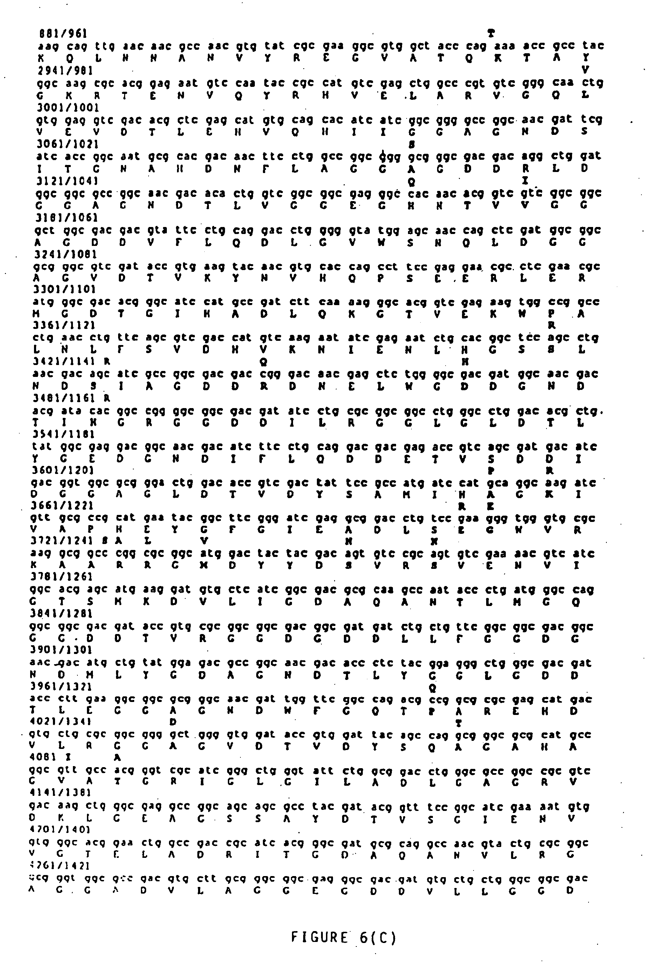

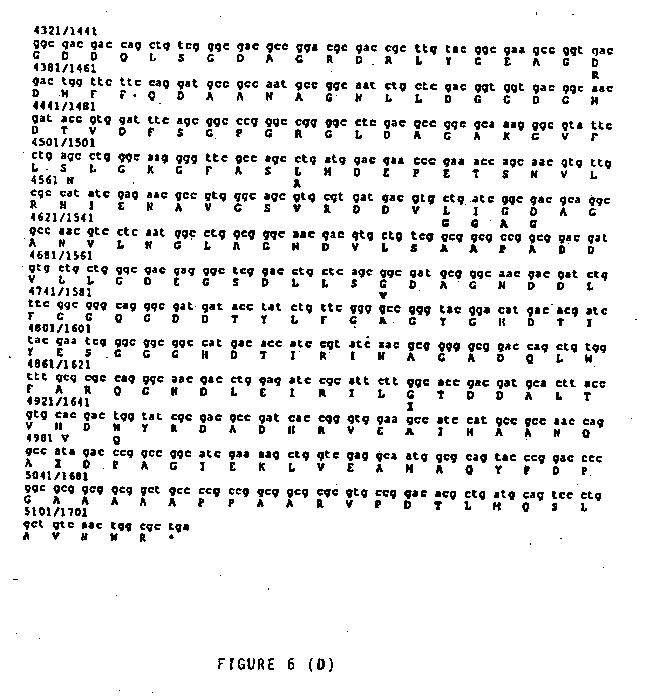

Par ailleurs, les séquences nucléotidiques codant pour l'AC-Hly de B. pertussis et de B. bronchiseptica sont présentées respectivement dans les figures 5 et 6.

La séquence nucléotidique du gène CyaC activateur du gène CyaA a été décrite dans la publication de Barry E.M. et al (Journal of Bacteriology, Jan. 1991, p. 720-726).

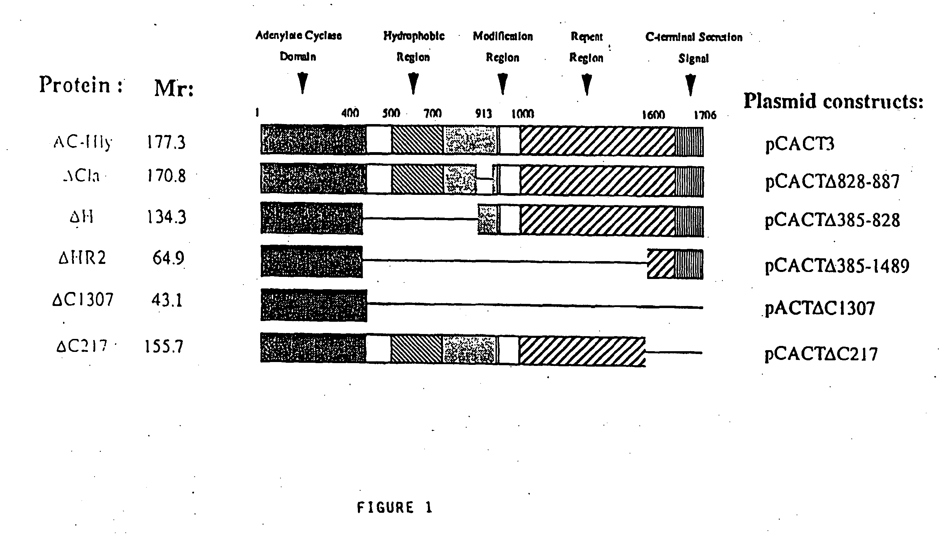

Une séquence intéressante dans le cadre de la présente invention est la séquence correspondant aux régions dites région modifiée et région répétée de l'AC-Hly (voir figure 1).

Lorsqu'une séquence antigénique hétérologue par rapport à la séquence polypeptidique de l'AC-Hly est présente, il peut s'agir d'une séquence d'un antigène d'un organisme pathogène bactérien, viral, parasitaire notamment, contre lequel on recherche par exemple la formation d'anticorps protecteurs.

Par l'expression « anticorps protecteurs contre une infection par B. pertussis et/ou B. parapertussis et/ou B. bronchiseptica, on entend des anticorps protégeant contre les infections létales et sublétales induites par ces bactéries, c'est à dire protégeant contre la maladie et l'infection.

Avantageusement, les séquences d'acides aminés selon l'invention, capables d'induire la formation d'anticorps protecteurs contre les infections ci-dessus désignées, sont associés à des facteurs impliqués dans la virulence des bordetella, utilisés par exemple sous forme de préparations polypeptidiques ou d'extraits bactériens ayant la capacité d'induire une protection contre la persistance des bactéries chez l'hôte.

Une séquence d'acides aminés intéressante dans le cadre de l'invention, est une séquence caractérisée en ce qu'elle est sous la forme d'un polypeptide ayant une conformation tridimensionnelle identique ou analogue à celle de la séquence polypeptidique correspondante de l'AC-Hly de B. pertussis ou de B. parapertussis ou de B. bronchiseptica, en ce qu'elle comprend l'enchaînement d'acides aminés situé entre les positions 900 environ, en particulier 910, et le dernier acide aminé C-terminal environ de la séquence polypeptidique de l'AC-Hly de B. pertussis ou d'une séquence correspondant à la précédente chez B. parapertussis ou chez B. bronchiseptica, ladite séquence comportant en outre une modification par addition d'un acide gras entre les acides aminés 980 environ et 985 environ, en particulier au niveau de l'acide aminé 983.

Cette séquence d'acides aminés comprend la région modifiée et la région répétée de l'AC-Hly.

Une autre séquence d'acides aminés préférée selon l'invention est une séquence caractérisée en ce qu'elle est formée par l'enchaînement des acides aminés compris entre l'acide aminé en position 385 environ et environ le dernier acide aminé C-terminal de la séquence polypeptidique de l'AC-Hly de B. pertussis ou d'une séquence correspondant à la précédente chez B. parapertussis ou chez B. bronchiseptica, ladite séquence comportant une modification par addition d'un acide gras entre les acides aminés 980 environ et 985 environ, de préférence au niveau de l'acide aminé 983.

La demande décrit également une séquence d'acides aminés, formée par l'enchaînement des acides aminés compris entre l'acide aminé en position 385 environ et l'acide aminé en position 400 environ de la séquence polypeptidique de l'AC-Hly de B. pertussis ou d'une séquence correspondant à la précédente chez B. parapertussis ou chez B. bronchiseptica.

Cette séquence d'acides aminés comprend avantageusement un épitope susceptible d'induire la formation d'anticorps protecteurs contre une infection par des Bordetella de type B. pertussis, B. parapertussis et B. bronchiseptica.

Est également décrite une séquence d'acides aminés, caractérisée en ce qu'elle est formée par l'enchaînement des acides aminés compris entre l'acide aminé en position 385 environ et l'acide aminé en position 500 environ de la séquence polypeptidique de l'AC-Hly de B. pertussis ou d'une séquence correspondant à la précédente chez B. parapertussis ou chez B. bronchiseptica.

Avantageusement, cette séquence comprenant les acides aminés 385 à 400 ou 385 à 500 est présentée dans une conformation identique ou analogue à la conformation qu'elle possède dans la protéine AC-Hly de Bordetella.

L'invention a également pour objet des séquences d'acides aminés obtenues par délétion de fragments polypeptidiques à partir de la séquence de l'AC-Hly de Bordetella, qu'il s'agisse de la séquence purifiée à partir de la bactérie, et en particulier à partir de B. pertussis, B. parapertussis ou B. bronchiseptica ou qu'il s'agisse d'une séquence obtenue à partir d'une protéine recombinante r-AC-Hly.

Une séquence d'acides aminés ainsi obtenue est avantageusement la séquence appelée ΔCla correspondant à l'enchaînement de l'AC-Hly modifié par délétion d'un fragment ΔCla représenté à la figure 1, correspondant à l'enchaînement des acides aminés 827 à 887 de la séquence polypeptidique de l'AC-Hly de B. pertussis ou d'une séquence correspondant à la précédente chez B. parapertussis ou chez B. bronchiseptica, la séquence obtenue comportant une modification par addition d'un acide gras entre les acides aminés 980 environ et 985 environ, de préférence au niveau de l'acide aminé 983.

Un autre fragment peut être délété de la séquence AC-Hly pour former une séquence d'acides aminés selon l'invention ; il s'agit du fragment polypeptidique ΔH correspondant à l'enchaînement des acides aminés 385 à 827 de la séquence polypeptidique de l'AC-Hly de B. pertussis ou d'une séquence correspondant à la précédente chez B. parapertussis ou chez B. bronchiseptica, la séquence obtenue comportant une modification par addition d'un acide gras entre les acides aminés 980 environ et 985 environ, de préférence au niveau de l'acide aminé 983.

L'invention a aussi pour objet la séquence d'acides aminés formant la « région répétée » de l'AC-Hly de Bordetella, comprise entre les résidus d'acides aminés 1000 et 1600 environ. La région répétée de B. bronchiseptica comporte une répétion de moins par rapport à l'AC-Hly de B. pertussis. A cet égard, l'invention concerne par exemple la séquence comprenant les acides aminés 1552 à 1592 de l'AC-Hly de B. pertussis.

L'invention concerne aussi les séquences nucléotidiques codant pour les séquences d'acides aminé de l'invention ci-dessus décrites.

Les gènes CyaA, CyaB et partiellement CyaD de B. bronchiseptica ont été clonés sur le plasmide pFBD2 hébergé par E. coli K12XL1 et déposé à la CNCM le 21 Juin 1995 sous le n° 1-1601.

Le plasmide pFBD2 est obtenu par insertion au site BamH1 du fragment de 8kb de B. bronchiseptica portant les gènes CyaA, CyaB et partiellement CyaD.

L'invention a par ailleurs pour objet des compositions polypeptidiques comprenant des séquences selon l'invention originaires de différents types de Bordetella. Par exemple, une composition polypeptidique avantageuse comprend une séquence définie précédemment de B. pertussis et une autre séquence ou plusieurs de ces séquences de B. parapertussis.

Une autre composition polypeptidique de l'invention comprend en outre une ou plusieurs séquences de B. bronchiseptica.

Selon un autre mode de réalisation de l'invention, une composition polypeptidique est caractérisée en ce qu'elle comprend une ou plusieurs séquence de l'invention définies précédemment de B. parapertussis et une ou plusieurs séquences de B. bronchisentica.

L'invention a également pour objet des composition immunogènes caractérisées en ce qu'elles comprennent une ou plusieurs des séquences de l'invention définies dans les pages précédentes.

De façon intéressante pour la protection contre l'infection par des Bordetella et en particulier pour la protection contre la persistance des bactéries chez l'hôte, une composition immunogène comprenant les séquences d'acides aminés de l'invention peut être caractérisée en ce qu'elle comprend en outre un extrait bactérien contenant les produits d'expression des gènes vrg d'une souche de Bordetella choisie parmi B. pertussis, B. parapertussis ou B. bronchiseptica ou une partie de ces produits d'expression, suffisante pour induire une réponse immunitaire chez un hôte auquel l'extrait serait administré.

Outre la présence de différentes adhésines et toxines, les Bordetelles sont caractérisées par une régulation de l'expression des facteurs impliqués dans leur virulence. Autrement dit, les Bordetelles subissent des variations et modulations de phase.

Les Bordetelles, suivant leur environnement, peuvent devenir "avirulentes" c'est à dire incapables d'induire la létalité, une réaction inflammatoire et des lésions pulmonaires dans le modèle murin d'infection respiratoire. Elles subissent soit une modulation de phase soit une variation de phase. La variation de phase s'observe à une fréquence allant de $10^{-3}$ à $10^{-6}$ et est quasiment irréversible. Elle se traduit par un arrêt de l'expression des toxines et adhésines décrites précédemment et par l'expression d'autres facteurs non encore bien caractérisés (changement des bactéries "virulentes" Phase I en bactéries "avirulentes" Phase IV). Les bactéries de phase I et de phase IV ont été décrites par Lacey B. 1960, J. Hyg, 58:57-93. La modulation de phase, phénotypiquement semblable à la variation de phase est totalement réversible et se traduit par un arrêt de la synthèse des adhésines et des toxines lors de changements environnementaux (composition du milieu de culture, température...).

La variation de phase et la modulation de phase observées chez Bordetella sont sous le contrôle de deux gènes régulateurs, bvg A et bvg S (Arico B. et al, 1989, Proc. Natl. Acad. Sci USA, 86: 6671-6675).

Le gène bvg S code pour une protéine sensible aux conditions extérieures. Cette protéine module par phosphorylation l'activité de la protéine codée par le gène bvgA, qui est d'une part un activateur positif de la transcription des gènes codant pour les facteurs de virulence (gènes vag pour "vir activated genes") cités précédemment (Uhl M. A. And Miller J. 1994. PRoc. Natl. Acad. Sci USA 91:1163-1167), d'autre part un répresseur de la transcription de certains gènes (Beattie D.T. et al, J. of Bacteriology, Jan 93, p. 159-527). Les gènes dont l'expression est réprimée sont appelés gènes vrg pour "vir repressed genes" et sont encore mal caractérisés. Il a cependant été montré que le gène vrg 6 de B. pertussis code pour une protéine ayant un rôle dans la persistance de la bactérie chez l'hôte (Beatties D. et al, 1992, Infect. Imm. 60:571-577). Chez B. bronchiseptica, deux protéines codées par les gènes vrg ont été caractérisées : ce sont des protéines de type flagelles (B. bronchiseptica Phase I est une bactérie immobile ne synthétisant pas de flagelles mais synthétisant adhésines et toxines et B. bronchiseptica Phase IV est une bactérie mobile synthétisant des flagelles).

La présence dans la composition immunogène, d'un extrait bactérien comprenant les produits d'expression des gènes vrg permettrait d'améliorer la réponse immunitaire humorale et/ou cellulaire obtenue après infection chez les sujets vaccinés et contribuerait également à la protection contre la persistance de la bactérie.

L'extrait bactérien dit "extrait bactérien vrg" dont question ci-dessus contient tous les constituants de la membrane externe d'une bactérie de phase IV, c'est à dire d'une bactérie n'exprimant pas les gènes vag, y compris l'endotoxine LPS. Cette endotoxine peut toutefois être éliminée ou détoxifiée.

Cet extrait peut être présent sous forme de suspension.

Les extraits bactériens « vrg » auxquels il est fait appel pour réaliser l'invention, sont de préférence des extraits dits « extraits urée ».

Un « extrait urée » est composé d'un mélange de protéines exprimées à la surface de la bactérie et qui sont séparées de la bactérie après incubation de celle-ci avec de l'urée 5M. L'extrait urée « vrg » contient, plusieurs protéines non encore caractérisées, les flagelles et le LPS.

L'utilisation d'extraits urée permet notamment la réalisation d'un vaccin de moindre coût par rapport à un vaccin qui serait obtenu à partir des protéines contenues dans les extraits, sous forme purifiée.

En outre, les inventeurs ont constaté que les extraits urée utilisés peuvent induire une réponse immunitaire cellulaire de type T (lymphoprolifération), se comportant ainsi comme le vaccin cellulaire utilisé jusqu'à ce jour.

Au contraire, les compositions exclusivement acellulaires n'induiraient pas de réponse T, réaction qui se produit pourtant en cas d'infection.

Les extraits urée vrg sont préparés respectivement à partir des bactéries de phase IV. Le cas échéant, les bactéries de phase IV sont remplacées par des bactéries dont le gène bvgS est muté de façon telle que les bactéries n'expriment que les protéines codées par les gènes vrg.

La préparation de ces extraits est décrite en détail dans la partie expérimentale.

Ainsi, l'invention concerne de façon préférée, une composition immunogène comprenant à la fois un extrait urée vag de B. pertussis et un extrait urée vrg de B. pertussis.

Une souche de B. pertussis appropriée pour la préparation de ces extraits est la souche HAV entrant dans le cadre de l'invention et déposée à la CNCM (Collection Nationale de Cultures de Microorganismes à Paris), le 19 Octobre 1994 sous le n° 1-1485. Pour préparer l'extrait urée vag, la souche HAV peut être utilisée directement puisqu'il s'agit d'une souche de phase I.

En revanche, l'extrait urée vrg est obtenu à partir d'une souche de phase IV dérivée de la souche de phase I par exemple par mutation du gène bvgS de la bactérie ou par culture de ladite souche de phase I dans un milieu contenant uniquement du sulfate de magnésium, de façon à obtenir l'expression des seuls gènes vrg de B. pertussis.

De la même manière et le cas échéant pour améliorer la réponse immunitaire chez l'hôte auquel on administrerait la composition immunogène, celle-ci comprend en outre une ou plusieurs adhésines ou toxines de B. pertussis, B. parapertussis ou B. bronchiseptica, choisie(s) parmi la FHA, les AGG ou la PRN et la PTX ou une partie de ces protéines, suffisante pour induire une réponse immunitaire chez un hôte auquel l'extrait serait administré.

De manière avantageuse, les séquences d'acides aminés dérivées de la toxine AC-Hly et le cas échéant les protéines exprimées par les gènes VRG sont obtenues à partir de la souche HAV déposée à la CNCM sous le n° I-1485.

De même, les séquences d'acides aminés de B. parapertussis utilisées dans le cadre de l'invention sont obtenues à partir de la souche n° 1 déposée à la CNCM sous le n° I-1498

Par ailleurs, les séquences d'acides aminés de la souche B. bronchiseptica utilisées dans le cadre de l'invention peuvent être obtenues à partir de la souche 9.735 déposée à la CNCM sous le n° I-858.

Les références données ci-dessus des différentes souches de Bordetella sont indiquées soit pour donner accès à la séquence des protéines et le cas échéant pour reproduire cette séquence par synthèse chimique, soit pour obtenir les séquences d'acides aminés de l'invention par protéolyse des protéines de Bordetella, soit pour donner accès à l'ADN des souches et ainsi permettre la production des séquences d'acides aminés de l'invention par les techniques du génie génétique.

L'invention a également pour objet une composition de vaccination comprenant à titre de principe actif une composition immunogène définie ci-dessus, en association avec un véhicule pharmaceutiquement acceptable et le cas échéant avec un adjuvant.

L'invention concerne également un procédé de préparation d'anticorps monoclonaux reconnaissant l'AC-Hly de B. pertussis, et l'AC-Hly de B. parapertussis et l'AC-Hly de B. bronchiseptica comprenant les étapes de :

- immunisation d'un animal, par exemple une souris Balb/c avec un peptide comprenant une séquence d'acides aminés de l'invention décrite ci-dessus , l'immunisation étant le cas échéant réalisée au moyen d'administrations répétées du peptide ;
- fusion des cellules spléniques de l'animal immunisé avec des cellules de myélome pour former un hybridome ;
- culture de l'hybridome dans des conditions permettant la production d'anticorps ;
- récupération des anticorps dirigés contre la séquence des acides aminés utilisée pour l'immunisation.

La demande décrit également un procédé de préparation d'anticorps monoclonaux reconnaissant l'AC-Hly de B. pertussis, et l'AC-Hly de B. parapertussis et l'AC-Hly de B. bronchiseptica comprenant les étapes de :

- immunisation d'un animal, par exemple une souris Balb/c avec un peptide comprenant la séquence des acides aminés 385 à 400 de l'AC-Hly de B. pertussis, l'immunisation étant le cas échéant réalisée au moyen d'administrations répétées du peptide ;
- fusion des cellules spléniques de l'animal immunisé avec des cellules de myélome pour former un hybridome ;
- culture de l'hybridome dans des conditions permettant la production d'anticorps ;
- récupération des anticorps dirigés contre la séquence des acides aminés 385 à 400 de l'AC-Hly de B. pertussis.

Le procédé précédemment décrit permet avantageusement, en utilisant pour l'immunisation un antigène spécifique de l'AC-Hly de B. pertussis d'obtenir des anticorps monoclonaux reconnaissant à la fois l'AC-Hly de B. pertussis ainsi que celles de B. parapertussis et de B. bronchiseptica. En outre, de tels anticorps monoclonaux ont de façon intéressante des capacités protectrices vis-à-vis de l'infection d'un hôte humain ou animal par des Bordetella de type B. pertussis, B. parapertussis et/ou B. bronchiseptica. Ainsi, les anticorps monoclonaux obtenus par mise en oeuvre du procédé précédemment décrit peuvent être utilisés pour le traitement de patients ou d'animaux infectés par une ou plusieurs des souches de Bordetella choisies parmi B. pertussis, B. parapertussis ou B. bronchiseptica.

De façon générale, l'invention a pour objet des anticorps monoclonaux caractérisés en ce qu'ils reconnaissent la séquence des acides aminés 385 à 400 de l'AC-Hly de B. pertussis.

L'invention concerne à ce titre des anticorps monoclonaux reconnaissant un épitope comprenant la séquence des acides aminés 385 à 400 de l'Ac-Hly de B. pertussis, produits par l'hybridome B5-4 déposé à la CNCM le 19 Juin 1996 sous le n° I-1734.

D'autres anticorps monoclonaux avantageux sont produits contre la séquence des 217 derniers acides aminés de l'AC-Hly de B. pertussis et sont produits par l'hybridome E17-21 déposé à la CNCM le 19 Juin 1996 sous le n° I-1733.

L'invention a également pour objet des anticorps monoclonaux dirigés contre l'une quelconque des séquences d'acides aminés précédemment décrites. Des anticorps monoclonaux dirigés spécifiquement contre la séquence C-terminale de l'Ac-Hly sont parmi les anticorps intéressants de l'invention. Des anticorps particuliers sont par exemple dirigés contre une séquence dérivée de l'enchaînement correspondant comportant les 217 derniers acides aminés de l'Ac-Hly de B. pertussis, notamment les acides aminés 1488 à 1705 de l'Ac-Hly de B. pertussis ou les acides aminés 1489 à 1706 de l'Ac-Hly de B. bronchiseptica. Ces anticorps monoclonaux peuvent être préparés par un procédé analogue à celui qui a été décrit ci-dessus pour les anticorps monoclonaux obtenus contre l'épitope contenu dans la séquence des acides aminés 385 à 400 environ de l'AC-Hly de B. pertussis.

Les anticorps de l'invention peuvent être humanisés par exemple en remplaçant la partie hypervariable d'une immunoglobuline humaine n'ayant pas une fonction anticorps, par une région hypervariable d'une immunoglobuline monoclonale obtenue au moyen de la technique décrite ci-dessus.

Des techniques permettant d'humaniser les anticorps ont par exemple été décrites par Waldmann T., Juin 1991, Science, vol. 252, p. 1657-1662 ; Winter G. et al, 1993, Immunology Today, vol. 14, N° 6, p. 243-246 ; Carter et al, Mai 1992, Proc. Natl. Acad. Sci. USA, vol. 89, p. 4285-4289 ; Singer et al, 1er Avril 1993, Journal of Immunology, vol. 150, n° 7, p. 2844-2857.

La demande décrit également des sérums polyclonaux tels qu'obtenus par immunisation d'un animal avec une séquence d'acides aminés répondant aux définitions données précédemment et récuparation des anticorps formés capables de reconnaître les séquences utilisées pour l'immunisation.

Le cas échéant, l'immunisation comporte l'administration, avec les séquences d'acides aminés, d'un adjuvant.

Selon un mode de réalisation particulier de l'invention, l'immunisation est réalisée avec une ou plusieurs séquences d'acides aminés et avec une composition immunogène comprenant des antigènes différents de l'AC-Hly.

L'invention concerne aussi une composition pharmaceutique comprenant à titre de principe actif, des anticorps monoclonaux selon l'invention.

Ces anticorps, en particulier les anticorps dirigés contre la séquence comprenant les acides aminés 385 à 400 et/ou les anticorps dirigés contre la partie C-terminale de la protéine Ac-Hly, peuvent être utilisés comme médicament en immunothérapie chez un hôte infecté par B. pertussis et/ou B. parapertussis et/ou B. bronchiseptica.

On aura par exemple recours aux anticorps monoclonaux produits par l'hybridome B5-4 déposé à la CNCM sous le n° I-1734 ou par l'hybridome E17-21 déposé à la CNCM sous le n° I-1733.

Les anticorps selon l'invention peuvent aussi être utilisés comme moyens d'analyse des souches de bordetelles collectées. Les anticorps monoclonaux dirigés contre la séquence d'acides aminés 385 à 400 ou contre la séquence C-terminale de la protéine Ac-Hly sont ainsi appropriés pour l'analyse des souches de B. pertussis.

Entre également dans le cadre de l'invention un procédé de détection in vitro d'une infection par une Bordetella notamment par B. pertussis, B. parapertussis ou B. bronchiseptica, caractérisé en ce que l'on met en contact un échantillon fluide biologique d'un patient ou d'un animal susceptible d'être infecté par Bordetella, avec des anticorps monoclonaux de l'invention ci-dessus définis et en ce que l'on détecte une réaction immunologique entre lesdits anticorps monoclonaux et les bactéries du genre Bordetella lorsqu'elles sont présentes dans l'échantillon.

La détection de l'infection par une bactérie du genre Bordetella, notamment par B. pertussis, B. parapertussis, B. bronchiseptica peut également être opérée sur un échantillon d'un fluide biologique d'un patient ou d'un animal par mise en contact de l'échantillon testé avec une séquence d'acides aminés de l'invention, suivie de la détection d'une réaction immunologique entre lesdites séquences d'acides aminés et des anticorps présents dans l'échantillon testé.

L'invention concerne aussi une composition pharmaceutique comprenant à titre de principe actif, des séquences nucléotidique de l'invention décrites précédemment.

L'utilisation des séquences nucléotidiques dans des compositions pharmaceutiques peut être réalisée par référence à la technique décrite par Donnelly et al (Nature Médecine, 1995, vol. 1, n° 6, p. 583-587).

FIGURES

Figure 1 : Représentation schématique des protéines tronquées de l'AC-Hly utilisée : La construction des plasmides et la production des protéines tronquées correspondantes a été décrite dans les publication antérieures de Sebo P. et al, 1991, Gene, 104:19-24 et Sebo P. et al, 1993, Mol. Microb. 9:999-1009. Les nombres placés dans la colonne « Mr » indiquent les masses moléculaires relatives des toxines, calculées à partir de leurs séquences d'acides aminés déduites. Les nombres qui suivent le symbole Δ dans le nom du plasmide sont les nombres du premier et du dernier acide aminé des parties délétées du cadre de lecture de cyaA. Les nombres qui suivent le symbole ΔC représentent les résidus C-terminaux manquants. Les allèles cyaA sont coexprimés avec le gène cyaC sous le contrôle de signaux d'initiation de transcription et de traduction du gène lacZ identique à celui présent sur le plasmide pCACT3 (Betsou F. et al, 1993, Infect. Immun. 61:3583-3589). ΔC1307 a été produit à partir de pDIA 5240 (pACTΔC1307) en présence du gène cyaC exprimé en trans à partir d'un plasmide compatible pPS4C (Betsou F. et al, 1993, Infect. Immun. 61:3583-3589 et Sebo P. et al, 1991, Gene, 104:19-24).

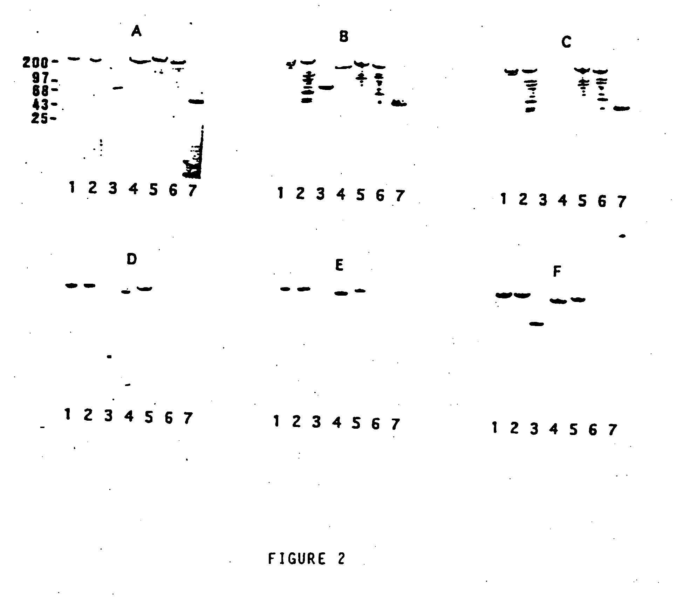

Figure 2 : Caractérisation de B. pertussis et de la protéine r-AC-Hly : 200 ng de B. pertussis purifiée et de r-AC-Hly ont été soumis à une électrophorèse sur SDS-PAGE 8-25% et les protéines ont été colorées avec du bleu de Coomassie (A) ou transformées sur une membrane HYBOND C-Super et incubées avec des sérums mélangés anti r-AC-Hly de 20 souris, 22 jours après la vaccination (B) ou avec des anticorps monoclonaux spécifiques de l'AC-Hly de B. pertussis (C) ou un mélange de sérums d'enfants infectés (infections confirmées par culture) (D) ou un sérum de souris infectées par B. pertussis 18323 collecté deux semaines après l'infection (E) ou un sérum de souris infectées par B. pertussis collecté deux mois après l'infection (F). L'immunodétection a été réalisée avec des anticorps anti-souris de lapin marqués avec la péroxydase. Ligne 1 : B. pertussis AC-Hly ; Ligne 2 : r-AC-Hly ; Ligne 3 : ΔHR2 ; Ligne 4 : ΔH ; Ligne 5 : ΔCla ; Ligne 6 : ΔC217 ; Ligne 7 : ΔC1307 les nombres indiquent les marqueurs de poids moléculaire.

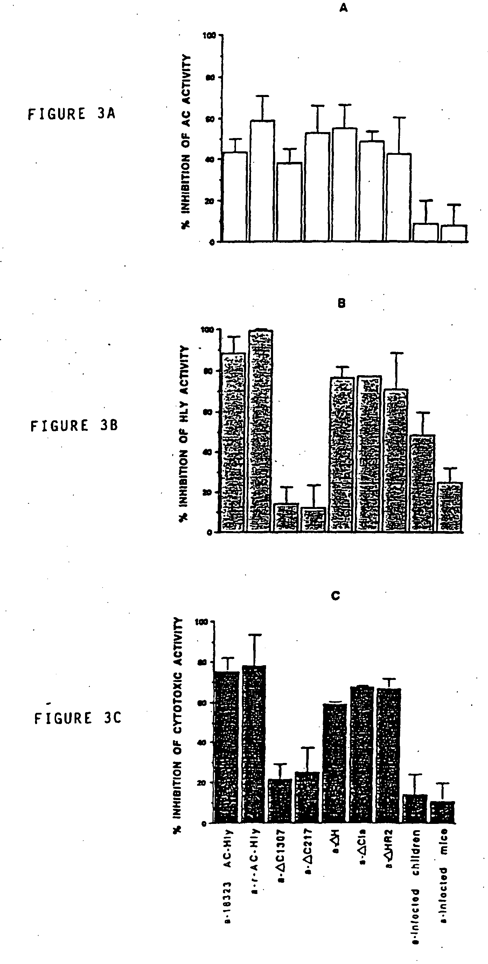

Figure 3 : Inhibition des activités adénylate cyclase (A), hémolytique (B) et cytotoxique (C) de l'AC-Hly de B. pertussis par des antisérums dirigés contre des fragments de la r-AC-Hly obtenus chez différentes souris et par des sérums de souris et d'enfants infectés par B. pertussis : l'AC-Hly a été incubée avec différents sérums comme décrit dans la partie matériels et méthodes. Les barres indiquent la déviation standard (n=4).

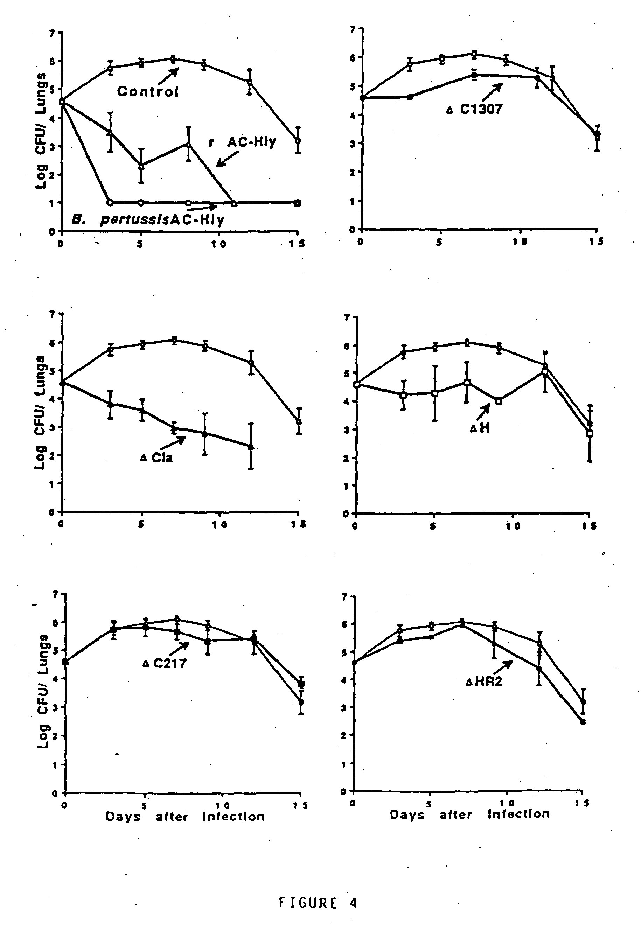

Figure 4 : Activités protectrices de la r-AC-Hly et de ses protéines tronquées. Des souris âgées de 3 à 4 semaines ont été immunisées deux fois, à deux semaines d'invervalles, avec 15μg de r-AC-Hly (Δ-Δ), ΔCla (▲-▲), ΔC1307 (●-●), ou ΔHR2 (□-□), ΔC217 (■-■), ΔH (□-□) ou B. pertussis AC-Hly ((O-O) adsorbées sur l'hydroxide d'aluminium, ou avec un tampon contenant de l'hydroxide d'aluminium seul comme contrôle (□-□). Elles sont infectées par voie intranasale deux semaines plus tard avec $10^5$ CFU de B. pertussis 18323. Les courbes montrent la déviation géométrique standard (barres) pour six souris par point.

Figure 5 : Séquence nucléotidique du gène codant pour l'AC-Hly de B. pertussis et séquence d'acides aminés correspondante.

Figure 6 : Séquence nucléotidique du gène codant pour l'AC-Hly de B. bronchiseptica et séquence d'acides aminés correspondante.

## EXEMPLES

## MATERIELS ET METHODES

### Souches bactériennes, plasmides et conditions de croissance:

La souche virulente B. pertussis 18323 (ATCC 9797) a été cultivée sur un milieu d'agar Bordet Gengou complété avec 15% de sang de mouton défibriné (BG) à 36°C pendant 72 heures et à nouveau pendant 24h. Des sous-cultures dans un milieu liquide ont été effectuées dans un milieu Stainer-Scholte (Stainer D.W. et Scholte M.J., 1971, J. Gen. Microb., 63:211-220) pendant 20 h à 36°C jusqu'à obtenir une densité optique à 650 nm de 1,0 (D.O.$_{620}$=1.0.).

Les plasmides utilisés pour la production de la protéine recombinante r-AC-Hly et de ses dérivés tronqués dans E. coli ont été décrits dans les publications suivantes : (Betsou F., P. Sebo, et N. Guiso, 1993, CyaC-mediated activation is important not only for toxic but also for protective activities of Bordetella pertussis adenylate cyclase-hemolysin, Infect. Immun. 61:3583-3589 ; Iwaki M., Ullmann A. et P. Sebo, 1995, « Oligomerization of the adenylate cyclase toxin of Bordetella pertussis : Evidence from in vitro complementation of individually inactive mutants ». Submitted.; et Sebo P., P. Glaser, H. Sakamoto et A. Ullmann, 1991, High Level synthesis of adenylate cyclase toxin of Bordetella pertussis in a reconstructed Escherichia coli system, Gene 104: 19-24.

Les plasmides permettent la production des différentes protéines (figure 1) en présence de la protéine activatrice CyaC. Des souches E. coli XL-1 Blue (Stratagène) comportant les plasmides respectifs ont été cultivés à 37°C sur un milieu 2xYT contenant 100 μg/L ampicilline à une densité optique D.O.$_{600}$ de 0,5 à 0,7, et induits pour obtenir la production de l'AC-Hly par de l'IPTG (1mm) pour quatre heures supplémentaires (Betsou F., P. Sebo, et N. Guiso, 1993, Infect. Immun. 61:3583-3589).

### Tests des activités adenylate cyclase, hémolytique et cytotoxique

L'activité adénylate cyclase a été mesurée selon le protocole décrit par Ladant D., C. Brezin, I. Crenon, J.M. Alonso, et N. Guiso, (1987, Bordetella pertussis adenylate cyclase: purification, characterization and radioimmunoassay, J. Biol. Chem. 261:16264-16269). Une unité (U) d'activité adénylate cyclase correspond à 1nmol de cAMP formé par minute à 30°C à pH 8,0. Les activités hémolytiques et cytotoxiques de l'AC-Hly ont été déterminées à 37°C, en utilisant des érythrocytes lavés de mouton (109/ml) selon la description de Bellalou J., H. Sakamoto, D. Ladant, C. Geoffroy et A. Ullmann, (1990, Deletions affecting hemolytic and toxin activities of Bordetella pertussis adenylate cyclase infect. Immun. 58: 3242-3247). Les concentrations en protéines ont été déterminées par la méthode de Bradford (A rapid and sensitive method for the quantification of micrograms of protein, utilising the principle of protein-dye binding. Anal. Biochem. 72:248-257).

### Tests d'inhibition de l'adénylate cyclase

L'AC-Hly purifiée de B. pertussis a été incubée à 100 U/ml dans 50 mM Tris-HCl à pH 7,6, avec 0,2 mM de Cacl$_2$ et 0,1 NP-40 avec différents sérums dilués 100 fois pendant 18 à 20 h à 4°C ; l'activité adénylate cyclase post incubatoire (IA) des échantillons a été mesurée. L'activité AC (adénylate cyclase) après incubation avec le sérum de souris immunisées avec de l'hydroxide d'aluminum uniquement (CA) a été prise comme référence d'activité 100% (autrement dit 0% d'inhibition).

Le pourcentage d'inhibition de l'activité AC a été calculé comme suit :

$$\% \text{ d'inhibition} = 100\% - (100\% \times \text{IA/CA}).$$

Tests d'inhibition de l'activité hémolytique

Une unité U de toxine et 5$\mu$l des différents sérums ont été mélangés dans 1ml de Tris-HCL 10 mM à pH 8, avec 2mM de CaCl$_2$, 150 mM de NaCl et 1$\mu$M de calmoduline bovine de cerveau et préincubés pendant 20 min à 4°C. Des érythrocytes lavés de mouton (10$^9$) ont été ajoutés et l'activité hémolytique restante a été déterminée après incubation pendant 3 h à température ambiante. Les érythrocytes non lysés ont été récupérés sous forme d'un culot après centrifugation à 2000 RPM et la densité optique de l'hémoglobine libérée (RH) dans les surnageants a été mesurée à 541 nm. L'activité hémolytique de la toxine incubée avec du sérum de souris immunisées uniquement avec l'hydroxide d'aluminium (CH) a été prise comme référence d'activité 100% (0% d'inhibition). Les érythrocytes de mouton incubés sans la toxine ont été utilisés comme contrôle de la lyse non-spécifique. Le pourcentage d'inhibition de l'activité hémolytique a été calculé comme suit : % d'inhibition = 100% - (100%xRH/CH).

Tests d'inhibition de l'activité cytotoxique

Une unité U de toxine et 5 $\mu$l de différents sérums ont été préincubés à 4°C pendant 20 minutes dans un volume total de 1 ml de Tris-HCL 10 mM à pH8, avec 2mM CaCl$_2$, 150 mM NaCl, 5mM Glucose, 1 mg/ml BSA et 1$\mu$M de calmoduline bovine de cerveau. Puis 10$^9$ érythrocytes lavés de mouton ont été ajoutés et l'incubation a été poursuivie à 37°C pendant 30 minutes. Pour arrêter l'activité de la toxine, 50$\mu$l de suspension d'érythrocytes ont été injectés dans 1ml d'acétate de sodium 50mM à ébullition à pH 5,2 et chauffés à 100°C pendant 5 min. La quantité de cAMP formée dans les érythrocytes lysés (IT) a été déterminée par une méthode ELISA standard (Pradelles P. Grassi J. Chabardes D., Guiso N. 1989, Analytical Chemistry, 61:447-450). La toxine incubée à 4°C a été utilisée comme contrôle négatif de la cytotoxicité. L'activité de la toxine incubée avec le sérum de souris immunisées avec l'hydroxide d'aluminium seulement (CT) a été considérée comme étant l'activité 100% (0% d'inhibition). Le pourcentage d'inhibition de l'activité cytotoxique a été calculé comme étant : % d'inhibition = 100% - (100%xIT/CT).

Méthodes d'électrophorèse et d'immunoblotting

Une électrophorèse SDS-PAGE a été réalisée avec un gel de polyacrylamide 8-25% prêt à l'utilisation pour le système PhastSystem (Pharmacia) et les protéines séparées ont été électrotransférées à partir des gels de polyacrylamide vers des membranes Hybond C-Super (Amersham). Après blocage, les membranes ont été incubées à une dilution de 10$^{-3}$ avec des sérums polyclonaux à 4°C pendant toute la nuit. La détection immunochimique a été réalisée en utilisant des immunoglobulines de mouton anti-souris marquées à la peroxydase de raifort et un système de chimioluminescence amélioré (ECL-Amersham).

Production et purification de l'AC-Hly

L'AC-Hly de B. pertussis, la r-AC-Hly (AC-Hly recombinante) produite à partir de E. coli et les différentes protéines tronquées recombinantes ont été extraites de bactéries avec de l'urée et purifiées sur des colonnes d'affinité à la calmoduline selon la méthode décrite par Guiso N., M. Szatanik et M. Rocandourt (1991, Protective activity of Bordetella adenylate cyclase against bacterial colonization. Microb. Pathog. 11:423-431). Les préparations enzymatiques ont été conservées dans de l'urée 8M avec du Tris-HCL 50 mM à pH 8, avec 0,2 mM CaCl$_2$, à -20°C. Toutes les préparations ont été analysées pour déterminer leur degré de pureté par électrophorèse sur gel de polyacrylamide SDS (SDS-PAGE).

Immunisation active

Les préparations purifiées de r-AC-Hly et des protéines tronquées ont été adsorbées à 60$\mu$g/ml sur de l'hydroxide d'aluminium (250 $\mu$g/ml). Pour réaliser une immunisation active, des souris Balb/c femelles âgées de 3 à 4 semaines (CERJ, St Berthevin, France) ont été traitées par injection par voie sous cutanée de 15 $\mu$g d'antigènes protéiques deux fois, à deux semaines d'intervalle. Les témoins ont reçu uniquement du tampon avec de l'hydroxide d'aluminium. Les souris ont ensuite été saignées une semaine suivant la dernière injection, de façon à permettre l'accès aux anticorps circulant présents. L'infection respiratoire sublétale a été réalisée deux semaines après la deuxième immunisation.

Infection intranasale des souris

La souche B. pertussis a été cultivée sur un milieu Bordet Gengou BG pendant 48h selon la description précédente et les bactéries ont été resuspendues dans 1 % de casaminoacides. Des quantités sublétales ont été administrées par injections intranasales de 50μl des suspensions bactériennes. Les souris infectées ont été sacrifiées par dislocation cervicale 1 heure après l'exposition (à un moment désigné par « Jour 0 ») et à différents jours après le jour 0 (6 souris lors de chaque étape). Les poumons ont été prélevés de façon asseptique et homogénéisés dans du sel avec un broyeur de tissus. Des dilutions des préparations homogènes de poumon ont été échantillonnées sur un milieu BG et des unités formant des colonies (cfu) ont été comptées après 3 jours d'incubation à 36°C. Toutes les expériences ont été réalisées au moins 2 fois et ont donné des résultats cohérents.

Préparation des sérums immuns

Pour obtenir les sérums des souris infectées, 10 souris Balb/c fermelles (âgées de 4 semaines) ont été infectées par voie intranasale avec $2 \times 10^5$ bactéries virulentes B. pertussis selon la technique décrite par Guiso N. Rocancourt, M. Szatanik et J.M. Alonso, (1989, Bordetella adenylate cyclase is a virulence associated factor and a protective antigen, Molec. Pathog. 7:373-380). Les souris ont été saignées 14 jours après l'infection ou à différents jours indiqués suivant ces 14 jours.
Les sérums polyclonaux de souris dirigés contre les dérivés tronqués de l'AC-Hly ont été collectés une semaine après la seconde injection faite aux souris avec chacun des antigènes considérés.
Le sérum de patients infectés a été préparé par réalisation d'un mélange de sérums immuns polyclonaux de 10 enfants non vaccinés sélectionnés, infectés par B. pertussis. Ces enfants étaient âgés de plus de 8 mois, pour exclure chez eux la présence d'anticorps maternels, et âgés de moins de 2 ans de façon à être certains de leur passé clinique.

RESULTATS

Propriétés immunologiques de la protéine r-AC-Hly et de ses variants tronqués

Il a été montré par le passé que la modification de l'AC-Hly intervenant grâce à l'intermédiaire de la protéine CyaC de B. pertussis est essentielle pour son activité protectrice (Betsou et al, CyaC-mediated activation is important not only for toxic but also for protective activities of Bordetella pertussis adenylate cyclase-hemolysin, Infect. Immun, 61:3583-3589). Il était donc important de déterminer si cette modification contribuait elle-même à la formation de l'épitope protecteur dans sa forme linéaire ou si cette modification induisait une modification conformationnelle dans la toxine, modification requise pour la présentation des épitopes protecteurs. Aussi, les propriétés immunologiques et protectrices d'un certain nombre de protéines tronquées ont-elles été examinées ; les protéines tronquées en question sont représentées schématiquement à la figure 1.
Ces protéines ont été produites dans E. coli en présence de la protéine CyaC en utilisant les plasmides pCACT ou pDIA afin de permettre l'acylation par CyaC au moyen des chaînes d'acides gras, des constructions comportant le site de modification. Comme le montre la figure 2A, les préparations purifiées de toutes les protéines contenaient un polypeptide majeur correspondant au poids moléculaire attendu. Ces protéines ont été contrôlées par un test en Western Blot pour vérifier leur reconnaissance par différents sérums. Comme le montre la figure 2B, le sérum produit contre la protéine r-AC-Hly reconnaissait la protéine r-AC-Hly et reconnaissait aussi la protéine AC-Hly purifiée à partir de B. pertussis ainsi que toutes les formes tronquées dérivées de r-AC-Hly. Conformément à la figure 2A, les préparations polypeptidiques purifiées comprenant la longueur totale de la protéine, en d'autres termes les préparations de AC-Hly, r-AC-Hly et les polypeptides tronqués ΔCla et ΔC217, contenaient également plusieurs fragments reconnus par un sérum polyclonal obtenu contre la protéine purifiée r-AC-Hly (figure 2B). Des anticorps monoclonaux (figure 2) préparés spécifiquement contre le domaine adénylase-cyclase de l'AC-Hly reconnaissaient également ces fragments de l'AC-Hly, ΔCla et ΔC217, montrant que ces fragments étaient des fragments protéolytiques tronqués dans la partie C-terminale et contenant le domaine AC qui co-purifiait avec l'AC-Hly sur calmoduline-agarose. Cependant, l'anticorps monoclonal ne reconnaissait pas les protéines ΔH ou ΔHR2 qui étaient dépourvues des résidus 385 à 828 et 1489 à 1706 respectivement, mais cet anticorps reconnaissait la protéine ΔC1307. En utilisant notamment ces observations, les inventeurs ont établi que la région de la molécule localisée entre les acides aminés 385 et 400 participait à la formation d'un épitope.
De façon tout à fait remarquable et au contraire de ce qui pouvait se passer avec le sérum anti-r-AC-Hly, les sérums obtenus chez des enfants infectés et utilisés en mélange n'ont pas reconnu le domaine AC de l'AC-Hly (ΔC1307 ; ligne 7), et n'ont pas reconnu non plus la protéine dépourvue des 217 résidus terminaux de l'AC-Hly (ΔC217 ; ligne 6), ni la protéine contenant les derniers 217 résidus mais dépourvue des régions hydrophobes, modifiée et de la majeure partie de la région répétée de l'AC-Hly (ΔHR2, ligne 3). Cependant ces sérums humains reconnaissaient l'AC-Hly de B. pertussis et la r-AC-Hly (figure 2D, lignes 1 et 2) et les deux protéines tronquées possédant des régions modifiée et répétée (derniers 900 résidus) de l'AC-Hly (figure 2D, lignes 4 et 5). Un schéma de reconnaissance identique (figure 2E) a été obtenu avec le sérum de souris infecté avec B. pertussis 18323 (souche de référence) et collecté rapidement après l'infection (14 jours). Les sérums collectés longtemps après l'infection (35 jours) reconnaissaient la partie C-terminale de l'AC-Hly présente sur la protéine ΔHR2 (figure 2F, ligne 3), alors qu'ils continuaient de ne pas reconnaître le domaine AC (figure 2F, ligne 7) et la protéine ΔC217 dépourvue uniquement des 217 derniers résidus (figure 2F, ligne 6). Ces résultats suggèrent fortement que les anticorps anti-AC-Hly synthétisés après l'infection par B. pertussis sont de façon prédominante dirigés contre la région C-terminale comportant la région de modification et la région répétée de l'AC-Hly (derniers 900 résidus). De plus, ni la protéine ΔC217 dépourvue des derniers 217 résidus de AC-Hly, ni la protéine ΔHR2 qui contenait les derniers 217 résidus n'ont été reconnues par les sérums immuns et murins suggérant que ces sérums polyclonaux reconnaissent une structure particulière de la région répétée de l'AC-Hly, structure qui est abolie par l'une quelconque des deux délétions non chevauchantes.

Il est important de remarquer que les sérums de patients ou de souris infectés reconnaissaient seulement les polypeptides s'étendant sur la longueur complète de la protéine (AC-Hly, r-AC-Hly, ΔCla et ΔH) mais pas leurs fragments protéolytiques. L'absence de reconnaissance par ces sérums des produits de protéolyse, qui contiennent le domaine AC et sont clivés dans leur partie C-terminale (voir plus haut la figure 2C), indique en outre que la région de modification et la région répétée de l'AC-Hly (900 derniers résidus) doivent être intactes pour être reconnues par ces sérums.

Inhibition des activités adénylate cyclase, hémolytique et cytotoxique par des sérums dirigés contre des dérivés tronqués de l'AC-Hly

On a recherché si des sérums de souris infectées par B. pertussis ou des sérums de patients humains infectés par B. pertussis ou encore des sérums polyclonaux dirigés contre les différentes formes tronquées de la rAC-Hly, obtenus par immunisation avec les protéines tronquées purifiées, pouvaient inhiber spécifiquement l'une des activités de la toxine. Comme un contrôle préalable à ces expériences était nécessaire, les titres ELISA des différents sérums ont été déterminés en utilisant la r-AC-Hly complète comme antigène de coating pour ces tests. Ces titres étaient similaires, à l'exception du titre obtenu avec le sérum anti-Δ C1307 qui ne reconnaissait pas l'AC-Hly en ELISA bien qu'il l'ait reconnu dans un test Western Blot comme les autres sérums. Comme le montre la figure 3A, l'activité adénylate cyclase a été inhibée par tous les sérums obtenus après immunisation de souris avec de la r-AC-Hly purifiée et avec des protéines dérivées tronquées. Cependant, aucun des sérums des souris infectées ni des patients humains infectés n'inhibait l'activité AC de l'AC-Hly dans des conditions identiques. Ceci est en accord avec le résultat obtenu par l'analyse Western blot (voir plus haut) montrant que ces sérums contiennent des anticorps dirigés de façon prédominante contre la partie C-terminale de l'AC-Hly (derniers 900 résidus) et non contre le domaine AC. En effet, l'activité hémolytique de la protéine AC-Hly était inhibée par les sérums d'enfants infectés et l'était moins fortement par les sérums de souris infectées. Cependant, une forte inhibition de l'activité hémolytique était observée avec des antisérums obtenus par immunisation avec les protéines AC-Hly, r-AC-Hly, ΔH, ΔCla et ΔHR2 de B. pertussis (figure 3B). Cette inhibition était probablement due à la présence d'anticorps spécifiques contre la partie C-terminale de AC-Hly. En fait, le sérum anti-ΔC1307 dirigé contre le domaine AC de l'AC-Hly qui était dépourvu de tels anticorps n'inhibait pas l'activité hémolytique de AC-Hly. De façon tout à fait intéressante, le sérum dirigé contre la protéine ΔC217 dépourvue uniquement des 217 derniers résidus n'inhibait pas non plus l'activité hémolytique de l'AC-Hly à un niveau mesurable. Ceci indique que les 217 derniers résidus sont soit la cible des anticorps neutralisants ou soit impliqués dans la formation d'une structure qui favorise la synthèse d'anticorps neutralisants dirigés contre d'autres parties de AC-Hly. Ensemble, ces résultats indiquent en outre que les anticorps synthétisés après l'infection sont principalement dirigés contre la partie hémolysine C-terminale de AC-Hly et sont capables de neutraliser l'activité hémolytique de cette protéine mais pas l'activité enzymatique de son domaine adénylate cyclase N-terminal. Comme le montre la figure 3C, une inhibition significative de l'activité cytotoxique de l'AC-Hly a été observée avec des sérums obtenus contre l'AC-Hly intacte et contre les protéines tronquées ΔH, ΔCla et ΔHR2 qui contiennent toutes à la fois le domaine AC et les 217 derniers résidus. Les antisérums ont aussi neutralisé les activités AC et hémolytiques. Au contraire, les 2 sérums qui inhibaient soit l'activité hémolytique seule (par exemple, les sérums de souris ou de patients infectés) soit l'activité AC seule (les sérums anti-ΔC1306 et anti-ΔC217, n'ont pas inhibé l'activité cytotoxique de l'AC-Hly. Ainsi, il apparaît que la présence des anticorps contre le domaine AC et contre les 217 derniers résidus de l'AC-Hly peut être requise pour neutraliser son activité cytotoxique.

Activité protectrice des différentes constructions d'AC-Hly recombinantes

Pour localiser les épitopes de l'AC-Hly requis pour obtenir une activité protectrice, l'activité protectrice de différentes protéines tronquées purifiées utilisant le modèle respiratoire murin a été testée. Des groupes de souris ont été immunisés deux fois avec l'hydroxide d'aluminium seul (témoin), ou avec des protéines tronquées purifiées adsorbées sur l'hydroxide d'aluminium puis ces souris ont été mises en contact par voie intranasale, avec des doses sublétales de souches B. pertussis 18323 virulentes. Ce modèle témoigne de la capacité des bactéries à adhérer, coloniser, survivre et se multiplier dans le système respiratoire des souris. Comme le montre la figure 4, les bactéries se multiplient rapidement dans les poumons des souris témoins pendant 6 jours après l'infection, puis commencent à être éliminées des poumons. Aucune multiplication des bactéries n'a été observée dans les poumons de souris immunisées avec les protéines AC-Hly ou r-AC-Hly de B. pertussis et après 3 jours, le nombre de bactéries a commencé à décroître (figure 4). En accord avec les observations antérieures (Betsou F., Sebo et N. Guiso, 1993, CyaC-mediated activation is important not only for toxic but also for protective activities of Bordetella pertussis adenylate cyclase-hemolysin, Infect. Immun. 61:3583-3589), l'efficacité protectrice de B. pertussis AC-Hly était plus élevée que celle de r-AC-Hly. Une protection similaire à celle induite par r-AC-Hly était aussi obtenue avec la protéine ΔCla, suggérant que la partie délétée dans la protéine ΔCla entre les résidus 827 et 887 n'était pas indispensable à l'induction de l'immunité protectrice. La protéine ΔH, manquant des résidus 385 à 828, présentait une activité protectrice plus faible que ΔCla. Aucune protection n'était induite par la protéine ΔC1307 dépourvue de la totalité de la partie hémolysine de l'AC-Hly. De façon plus intéressante, la protéine ΔC217 dépourvue des 217 derniers résidus et la protéine ΔHR2 contenant les derniers 217 résidus de l'AC-Hly, ne permettaient pas d'induire la protection. L'activité protectrice est donc corrélée avec le schéma de reconnaissance des constructions individuelles par des sérums de patients infectés ou de souris infectées.

DISCUSSION

Les anticorps dirigés contre l'AC-Hly sont habituellement présents dans des sérums d'enfants infectés (Arciniaga J.L., E.L. Hewlett, F.D. Jonhson, A. Deforest, S.G.F. Wassilak, I.M. Onorato, C.R. Manclark, et D.L. Burns, 1991, J. Infect. Dis. 163:135-142 ; et Guiso N., E. Grimprel, I. Anjak et P. Bégué, 1993, Eur. J. Clin. Microbiol. and Infect. Dis. In press) et il a été dans le passé démontré que l'immunisation avec l'AC-Hly protège des souris contre la colonisation bactérienne par Bordetella (Khelef, N., H. Sakamoto et N. Guiso, 1992, Microb. Pathog. 12:227-235). L'obtention d'un ensemble de formes tronquées de l'AC-Hly recombinante a permis de réaliser une étude de l'importance de différents domaines de la protéine dans l'induction de l'immunité protectrice par vaccination avec l'AC-Hly. Les résultats présentés ci-dessus montrent que les anticorps anti-AC-Hly, synthétisés après infection par B. pertussis sont de façon prédominante dirigés contre le domaine modifié et le domaine répété de l'AC-Hly (environ 800 résidus). En effet, seules les formes tronquées de l'AC-Hly possédant ces régions intactes étaient reconnues par des sérums de souris et de patients infectés dans un test Western-Blot et ces protéines étaient les seules à présenter la capacité d'induire la protection chez la souris. Ceci indique qu'au moins dans le cas de la souris, un ensemble relativement limité d'épitopes identiques ou se chevauchant pourrait être intéressant pour, à la fois l'induction de la synthèse d'anticorps anti-AC-Hly par les sujets infectés et pour l'induction de la synthèse d'anticorps protecteurs obtenus après vaccination avec l'AC-Hly. Les expériences rapportées précédemment ont montré que la suppression des 217 derniers résidus de l'AC-Hly qui sont en position distale par rapport aux sites de modification de la protéine par le produit d'expression du gène CyaC, au niveau du résidu lysine 983, abolissait la reconnaissance de cette protéine (ΔC217) par les sérums de souris et de patients infectés et abolissait également son activité protectrice. Cependant, ceci n'était pas dû à un défaut de modification de la protéine ΔC217 puisque cette protéine était acylée au niveau des chaînes d'acides gras lorsqu'elle était produite en présence de la protéine CyaC. De plus, les 217 résidus en soi n'étaient pas reconnus par les sérums et ne montraient pas d'activité protectrice lorsqu'ils étaient présentés dans la protéine ΔHR2 qui elle était dépourvue du site de modification (lysine 983) et d'une grande partie de la région répétée.

On en a conclu que à la fois les 217 derniers résidus et la région de modification était importants pour l'activité protectrice de AC-Hly. Ainsi, une interaction entre la région modifiée et les 217 derniers résidus de l'AC-Hly serait requise pour la formation ou l'activité des épitopes protecteurs de l'AC-Hly. De manière surprenante, ni la protéine ΔC217 manquante des 217 derniers résidus de l'AC-Hly, ni la protéine ΔHR2 dépourvue de la région modifiée et d'une grande partie des répétitions mais contenant les 217 derniers résidus, n'ont été reconnus par les sérums polyclonaux de patients infectés et/ou de souris (sérum frais). Les antisérums pourraient avoir une spécificité étroite contre un unique épitope, ou contre un petit groupe d'épitopes localisés au point de rupture des régions répétées présentes dans les protéines ΔC217 et ΔHR2 (proline 1489). Cependant, cette possibilité semble peu probable avec des antisérums polyclonaux mélangés. En outre, les sérums de souris et de patients humains infectés reconnaissaient la plupart des polypeptides de longueur complète des différentes protéines mais ne reconnaissaient pas les fragments protéolitiques C-terminaux clivés présents dans ces préparations, fragments qui étaient reconnus par un anticorps monoclonal anti-AC. Pris ensemble, ces résultats suggèrent que la formation d'épitopes protecteurs sur l'AC-Hly et la reconnaissance de l'AC-Hly par les sérums de sujets infectés nécessitent la présence d'une structure particulière formée seulement lorsque les régions modifiée et répétée de l'AC-Hly sont présentes. On peut concevoir que la formation de cette structure nécessiterait la modification par acylation des chaînes d'acides gras après la traduction de l'AC-Hly au niveau du résidu lysine 983 et pourrait être abolie par la suppression de la partie C-terminale de l'AC-Hly. A cet égard, il est important qu'à la fois les activités hémolytiques et cytotoxiques de l'AC-Hly soient perdues lorsque l'on supprime les derniers 75 résidus de l'AC-Hly qui contiennent le signal de sécrétion non modifié et ne soient pas directement impliquées dans l'activité de formation des pores (4 et 29). Ces observations supportent en outre l'hypothèse que la partie extrême de la région C-terminale de l'AC-Hly joue un rôle essentiel dans la structure globale de l'AC-Hly.

On a déjà suggéré que des épitopes protecteurs majeurs de l'AC-Hly seraient localisés dans la partie AC (Guiso, N., M. Rocancourt, M. Szatanik et J.M. Alonso, 1989, Molec. Pathog. 7:373-380 et Guiso, N. M. Szatanik, et M. Rocancourt, 1991, Microb. Pathog. 11:423-431). Les résultats présentés ici montrent que la contamination par des fragments AC-Hly s'étendant sur les régions modifiée et répétée de l'AC-Hly entrerait dans l'obtention d'une activité protectrice associée avec les fragments du domaine AC présent dans les préparations décrites dans l'état de la technique. On peut néanmoins douter de la capacité de ces contaminants en quantités mineures à produire une activité protectrice. Une autre interprétation serait que, dans certaines conditions, la région entre les acides aminés 385 et 450 ou 500 pourrait également jouer un rôle dans l'activité protectrice de l'AC-Hly. La structure de cette région pourrait être modifiée dans l'AC-Hly en l'absence des 217 derniers résidus d'acides aminés ou en l'absence de l'acylation. La structure de la partie N-terminale de 40-50 kDa et la présentation au système immunitaire pourrait induire la protection lorsque cette structure serait clivée par rapport au reste de la molécule d'AC-Hly dans les surnageants de culture de B. pertussis. Un argument de cette hypothèse serait qu'un anticorps monoclonal dirigé contre la partie de la molécule localisée entre les acides aminés 385 et 400 est un anticorps protecteur.

On a également examiné si une corrélation existe entre l'activité protectrice d'une protéine tronquée obtenue à partir de la r-AC-Hly in vivo et son activité à induire la synthèse d'anticorps qui pourrait neutraliser l'activité toxique in vitro. Une telle corrélation n'a pas été établie. Alors que toutes les protéines ayant une activité protectrice induisaient la synthèse d'anticorps neutralisants, la protéine ΔHR2 qui n'était pas du tout protectrice induisait une réponse anticorps fortement neutralisante. Ceci suggère que la présence d'anticorps neutralisants vis-à-vis de l'activité toxique de l'AC-Hly n'est pas une mesure fiable de l'induction de la protection contre l'infection par B. pertussis.

Il est par ailleurs important de noter que la protéine ΔCla, dépourvue d'une partie du domaine hydrophobe, présentait une activité protectrice. Cette protéine recombinante est un bon candidat pour l'inclusion dans des vaccins acellulaires contre pertussis puisqu'elle n'exerce pas d'activité cytotoxique résiduelle.

Préparation d'extrait urée vrg

Les extraits urée vrg sont préparés à partir des 3 mêmes espèces bactériennes phase IV c'est à dire à partir de bactéries n'exprimant plus aucun gène vag mais exprimant les gènes vrg. Les produits de ces gènes ne sont pas encore bien caractérisés, à l'exception des flagelles de B. bronchiseptica. Les extraits urée vrg se préparent comme les extraits urée vag.

Description des milieux de culture

Milieu Bordet Gengou deshydraté

Composition:

|  | 1 litre | 5 litres |
|---|---|---|
| Milieu Bordet Gengou | 30 g | 150 g |
| Glycérol | 10 ml | 50 ml |
| Eau pyrolysée | 1 litre | 5 litres |

Ajuster le pH à 7,4

- Faire chauffer
- Autoclaver 15 minutes à 120°
- Conserver à 4°C

Au moment de l'emploi :
Le Bordet Gengou est enrichi avec 15 à 20% de sang de mouton ou de cheval. Faire fondre les tubes et les maintenir fondus à 54°C. Rajouter 2,5 ml de sang de mouton dans chaque tube, stérilement. Verser le contenu du tube dans une boîte de pétri stérile.

Remarque:

Pour la recherche de <u>Bordetella pertussis</u> à partir de prélèvement nasopharyngé, utiliser des boîtes fraîches (maximum 7 jours à 4°C)

Milieu Gélose CSM

Pour préparer 2 litres d'une solution concentrée 10 fois :

| | | |
|---|---|---|
| Sodium Hydrogénoglutamate.. | (Réf. Prolabo n°27872.298) | 107g |
| L-Proline | (Réf. Merck n°7434) | 2,4g |
| NaCl | (Réf. Prolabo n°27810.295) | 25g |
| H2PO4 | (Réf. Prolabo n°26926.298) | 5g |
| KCl | (Réf. Prolabo n°26759.291) | 2g |
| MgCl2 | (Réf. Prolabo n°25108.295) | 1g |
| Tris-base | (Réf Merck n°8382.2500) | 15,2g |
| Casamino Acids | (Réf. Difco n° 0288-01-2) | 5g |
| Solution de CaCl2 à 1 % dans l'eau pyrolysée | (Réf. Prolabo n°22317.297 | 20 ml |
| Eau pyrolysée | QSP | 1 litre |

Dissoudre les différents constituants dans une partie du volume d'eau final. Ajuster le pH à 7,4 à l'aide d'acide chlorydrique. Compléter au volume final et conserver à -20°C.

- Au moment de l'emploi, mélanger:

  - 100 ml de la solution concentrée 10 fois
  - 900 ml d'eau pyrolysée
  - 1 g de (2,6-0-Dimethyl) cyclodextrine référence Aldrich n° 51166-71-3
  - 15 g de Bacto agar référence Difco n°0140-01

  Répartir par fractions de 20 ml en tube de verre
  Stériliser et ajouter le complément stérile.
- Solution de complément :
- Mélanger :

  - 1 ml de solution de complément concentrée 10 fois
  - 100 mg de glutathion référence Merck n° 4090
  - 9 ml d'eau pyrolysée

  Filtrer cette solution sur filtre millex 0,22 $\mu$m
  Ajouter 200 $\mu$l de cette solution à 1 tube de 20 ml de milieu

Milieu de culture Stainer

A. Milieu de Base

Pour préparer 2 litres d'une solution concentrée 10 fois :

| | | |
|---|---|---|
| Sodium Hydrogénoglutamate | (Réf. Prolabo n°27872.298) | 214,0g |
| L-Proline | (Réf. Merck n°7434) | 4,8g |
| NaCl | (Réf. Prolabo n°27810.295) | 50,0g |
| H2PO4 | (Réf. Prolabo n°26926.298) | 10,0g |
| KCl | (Réf. Prolabo n°26759.29 | 1 4,0g |
| MgCl2 | (Réf. Prolabo n°25108.295) | 2,0g |
| Tris-base | (Réf. Merck n°8382.2500) | 30,5g |

(suite)

| | | |
|---|---|---|
| Solution de CaCl2 à 1 % dans l'eau pyrolysée | (Réf. Prolabo n°22317.297 | 40 ml |
| Eau pyrolysée | QSP | 2 litres |

Dissoudre les différents constituants dans une partie du volume d'eau final. Ajuster le pH à 7,6 à l'aide d'acide chlorydrique. Compléter au volume final et répartir cette solution concentrée qui peut être conservée à -20°C pendant plusieurs semaines.

Au moment de l'emploi, diluer le milieu, le stériliser à 120°C pendant 15 minutes puis ajouter le complément stérilisé par filtration.

<u>B. Solution de complément</u>

Pour préparer 200 ml d'une solution concentrée 10 fois :

| | | |
|---|---|---|
| - L-Cystine | (Réf. Prolabo n°23260.184) | 8g |
| -HCl concentré | | 20ml |

Dissoudre. Sur cette préparation, verser le mélange suivant préalablement dissout :

| | | |
|---|---|---|
| $FeSO_4, 7H_2O$ | (Réf. Prolabo n°24244.232) | 2g |
| Acide L(+) ascorbique | (Réf. Prolabo n°20155.237) | 4g |
| Acide nicotinique | (Réf. Merck n°6817) | 0,8g |
| Eau pyrolysée | 120ml | |

Compléter à 200 ml avec de l'eau pyrolysée, répartir la solution par fractions de 1, 2, 3 ou 4 ml et congeler à -20°C.

Au moment de l'emploi, diluer la solution 10 fois dans l'eau pyrolysée et ajouter :

le glutathion (Réf. Merck n°4090) 100mg/10ml de complément dilué, stériliser cette solution par filtration (filtre Millex 0,22 $\mu$m à usage unique) et ajouter 1 ml de solution stérile à 100 ml de milieu de base stérile.

<u>2.1.2. Préparation de l'extrait urée vrg</u>

- Centrifuger la suspension bactérienne 30 minutes à 5000 g à 4°C.
- Resuspendre le culot bactérien dans de l'urée 5M préparée dans du tampon PBS (décrit plus loin) à raison d'un volume égal à 5 fois le poids humide du culot bactérien.
- Laisser sous agitation durant 1 heure à 4°C puis centrifuger 40 minutes à 40000 g à 4°C,
- Conserver le surnageant à -80°C jusqu'à utilisation.

<u>2.1.3.Inactivation de l'extrait urée vrg</u>

- Après élimination de l'urée, par passage sur colonne G25, l'extrait urée vrg est dilué en PBS de façon à obtenir une concentration protéique de 300 $\mu$g/ml.
- Ajouter goutte à goutte un volume de glutaraldéhyde à 2,5% de façon à obtenir une concentration finale de 0,05%.
- Laisser 2 heures à température ambiante en mélangeant régulièrement.
- Arrêter la réaction par addition de lysine (concentration finale 0,02M).
- Après 2 heures à température ambiante, l'extrait urée est adsorbé sur hydroxyde d'alumine préparé en PBS (1 mg/ml), pendant une nuit à 4°C sous agitation douce. Le vaccin est alors prêt pour l'immunisation de l'animal à raison de 10 à 20 $\mu$g/injection (décrit plus loin).

## Revendications

1. Séquence d'acides aminés dérivée de la séquence polypeptidique de l'adenylcyclase-hémolysine (AC-Hly), **caractérisée en ce qu'**elle est capable d'induire la formation d'anticorps protecteurs contre une infection par *B. pertussis* et/ou *B. parapertussis* et/ou *B. bronchiseptica* et **en ce qu'**elle est choisie parmi les enchaînements suivants :

   a) la séquence d'une protéine tronquée comprenant l'enchaînement des acides aminés situés approximativement entre la position 910, de préférence 913, et le dernier acide aminé C-terminal de la séquence polypeptidique de l'AC-Hly de *B. pertussis* ou d'une séquence correspondant à la précédente chez *B. parapertussis* ou chez *B. bronchiseptica,* la dite séquence comportant une modification par addition d'un acide gras entre les acides aminés 980 environ et 985 environ, de préférence au niveau de l'acide aminé 983 ;
   b) une séquence ayant une conformation tridimensionnelle identique ou analogue à celle de la séquence polypeptidique correspondante de l'AC-Hly de *B. pertussis, B. parapertussis* ou *B. bronchiseptica,* comprenant un enchaînement d'acides aminés tel que défini ci-dessus en a) et un enchaînement de 16 à 500 acides aminés comprenant une séquence consistant en l'enchaînement des acides aminés 385 à 400, 385 à 450, ou 385 à 500 de la séquence polypeptidique de l'AC-Hly de *B. pertussis* ou d'une séquence correspondant à la précédente chez *B. parapertussis* ou chez *B. bronchiseptica* ces deux enchaînements étant réunis par l'intermédiaire d'une séquence antigénique dérivée d'une protéine différente de l'AC-Hly.
   c) une séquence ayant une conformation tridimensionnelle identique ou analogue à celle de la séquence polypeptidique correspondante de l'AC-Hly de *B. pertussis, B. parapertussis* ou *B. bronchiseptica,* comprenant un enchaînement d'acides aminés tel que défini ci-dessus en a) et un enchaînement de 16 à 500 acides aminés comprenant une séquence consistant en l'enchaînement des acides aminés 385 à 400, 385 à 450, ou 385 à 500 de la séquence polypeptidique de l'AC-Hly de *B. pertussis* ou d'une séquence correspondant à la précédente chez *B. parapertussis* ou chez *B. bronchiseptica*, ces deux enchaînements étant soit contigus, soit réunis par l'intermédiaire de l'enchaînement d'acides aminés naturellement présent entre les deux enchaînements définis ci-dessus au sein de l'AC-Hly de *B. pertussis, B. parapertussis* ou *B. bronchiseptica,* la séquence d'acides aminés obtenue étant tronquée par rapport à la séquence Ac-Hly de *Bordetella.*

2. Séquence d'acides aminés selon la revendication 1 **caractérisée en ce qu'**elle est sous la forme d'un polypeptide ayant une conformation tridimensionnelle identique ou analogue à celle de la séquence polypeptidique correspondante de l'AC-Hly de *B. pertussis* ou de *B. parapertussis* ou de *B. bronchiseptica,* **en ce qu'**elle comprend l'enchaînement d'acides aminés situé entre la position 900 environ, en particulier 910, et le dernier acide aminé C-terminal environ de la séquence polypeptidique de l'AC-Hly de *B. pertussis* ou d'une séquence correspondant à la précédente chez *B. parapertussis* ou chez *B. bronchiseptica,* ladite séquence comportant en outre une modification par addition d'un acide gras entre les acides aminés 980 environ et 985 environ, en particulier au niveau de l'acide aminé 983.

3. Séquence d'acides aminés selon la revendication 1, **caractérisée en ce qu'**elle est formée par l'enchaînement des acides aminés compris entre l'acide aminé en position 385 environ et le dernier acide aminé C-terminal environ de la séquence polypeptidique de l'AC-Hly de *B. pertussis* ou d'une séquence correspondant à la précédente chez *B. parapertussis* ou chez *B. bronchiseptica,* ladite séquence comportant une modification par addition d'un acide gras entre les acides aminés 980 environ et 985 environ, de préférence au niveau de l'acide aminé 983.

4. Séquence d'acides aminés selon la revendication 1, **caractérisée en ce qu'**il s'agit de la séquence polypeptidique de l'AC-Hly modifiée par délétion d'un fragment polypeptidique appelé ΔCla correspondant à l'enchaînement des acides aminés 827 à 887 de la séquence polypeptidique de l'AC-Hly de *B. pertussis* ou d'une séquence correspondant à la précédente chez *B. parapertussis* ou chez *B. bronchiseptica,* la séquence obtenue comportant une modification par addition d'un acide gras entre les acides aminés 980 environ et 985 environ, de préférence au niveau de l'acide aminé 983.

5. Séquence d'acides aminés selon la revendication 1, **caractérisée en ce qu'**il s'agit de la séquence polypeptidique de l'AC-Hly modifiée par délétion d'un fragment polypeptidique appelé ΔCla correspondant à l'enchaînement des acides aminés 385 à 827 de la séquence polypeptidique de l'AC-Hly de *B. pertussis* ou d'une séquence correspondant à la précédente chez *B. parapertussis* ou chez *B. bronchiseptica*, la séquence obtenue comportant une modification par addition d'un acide gras entre les acides aminés 980 environ et 985 environ, de préférence au niveau de l'acide aminé 983.

6. Séquence d'acides aminés selon l'une quelconque des revendications 1 à 5, **caractérisée en ce que** la modification par addition d'un acide gras est une palmitoylation.

7. Composition polypeptidique, **caractérisée en ce qu'**elle comprend une séquence selon l'une quelconque des revendications 1 à 6 de *B. pertussis* et une séquence selon l'une quelconque des revendications 1 à 6 de *B. parapertussis*.

8. Composition polypeptidique selon la revendication 7, **caractérisée en ce qu'**elle comprend en outre une séquence selon l'une quelconque des revendications 1 à 6 de *B. bronchiseptica*.

9. Composition polypeptidique, **caractérisée en ce qu'**elle comprend une séquence selon une quelconque des revendications 1 à 6 de *B. parapertussis* et une séquence selon une quelconque des revendications 1 à 6 de *B. bronchiseptica*.

10. Séquence nucléotidique codant pour une séquence d'acides aminés selon l'une quelconque des revendications 1 à 5.

11. Composition immunogène **caractérisée en ce qu'**elle comprend une ou plusieurs séquences selon l'une quelconque des revendications 1 à 6.

12. Composition immunogène selon la revendication 11, **caractérisée en ce qu'**elle comprend en outre un extrait bactérien contenant les produits d'expression des gènes vrg d'une souche de Bordetella choisie parmi *B. pertussis, B. parapertussis* ou *B. bronchiseptica* ou une partie de ces produits d'expression, suffisante pour induire une réponse immunitaire chez un hôte auquel l'extrait serait administré.

13. Composition immunogène selon la revendication 11, **caractérisée en ce qu'**elle comprend en outre une ou plusieurs adhésines ou toxines de *B. pertussis, B. parapertussis* ou *B. bronchiseptica*, choisie(s) parmi la FHA, les AGG ou la PRN et la PTX ou une partie de ces protéines, suffisante pour induire une réponse immunitaire chez un hôte auquel l'extrait serait administré.

14. Composition immunogène selon l'une quelconque des revendications 11 à 13, **caractérisée en ce que** la souche de *B. pertussis* à partir de laquelle sont obtenues les séquences d'acides aminés dérivées de la toxine AC-Hly et le cas échéant les protéines exprimées par les gènes vrg, est la souche HAV déposée à la CNCM sous le n° I-1485.

15. Composition immunogène selon l'une quelconque des revendications 11 à 13, **caractérisée en ce que** la souche de *B. parapertussis* à partir de laquelle sont obtenues les séquences d'acides aminé dérivées de la toxine AC-Hly et les protéines exprimées par les gènes vrg, est la souche n° 1 déposée à la CNCM sous le n° I-1498.

16. Composition immunogène selon l'une quelconque des revendications 11 à 13, **caractérisée en ce que** la souche de *B. bronchiseptica* à partir de laquelle sont obtenues les séquences d'acides aminés dérivées de la toxine Ac-Hly et les protéines exprimées par les gènes vrg, est la souche 9.73 S déposée à la CNCM sous le n° I-858.

17. Composition vaccinante **caractérisée en ce qu'**elle comprend à titre de principe actif, une composition immunogène selon l'une quelconque des revendications 11 à 16, en association avec un véhicule pharmaceutiquement acceptable et le cas échéant un adjuvant.

18. Procédé de préparation d'un sérum polyclonal, comprenant les étapes de :

- immunisation d'un animal non-humain avec une ou plusieurs séquences d'acides aminés selon l'une quelconque des revendications 1 à 5 associées ou non à un adjuvant,
- récupération des anticorps formés, capables de reconnaître les séquences d'acides aminés utilisées pour l'immunisation.

19. Procédé de préparation selon la revendication 18, **caractérisé en ce que** l'immunisation de l'animal est réalisée avec une ou plusieurs séquences d'acides aminés selon l'une quelconque des revendications 1 à 7 et une composition immunogène comprenant des antigènes différents de l'AC-Hly.

20. Procédé de préparation d'anticorps monoclonaux reconnaissant l'AC-Hly de *B. pertussis* et/ou de *B. parapertussis* et/ou de *B. bronchiseptica,* comprenant les étapes de :

- immunisation d'un animal non-humain, par exemple une souris Balb/c avec une séquence d'acides aminés selon l'une quelconque des revendications 1 à 5,
- fusion des cellules spléniques de l'animal immunisé avec des cellules de myélome pour former un hybridome ;
- culture de l'hybridome dans des conditions permettant la production d'anticorps ;
- récupération des anticorps dirigés contre la séquence des acides aminés utilisée pour l'immunisation.

21. Anticorps monoclonaux **caractérisés en ce qu'**ils sont dirigés contre la séquence des acides aminés 385 à 400 de l'AC-Hly de *B. pertussis.*

22. Anticorps monoclonaux **caractérisés en ce qu'**ils sont dirigés spécifiquement contre la séquence des 217 derniers acides aminés de l'Ac-Hly de *B. pertussis,* notamment la séquence d'acides aminés 1488 à 1705 de l'Ac-Hly de *B. pertussis* ou la séquence d'acides aminés 1489 à 1706 de l'Ac-Hly de *B. bronchiseptica*.

23. Anticorps monoclonaux selon la revendication 21 ou 22 tels qu'obtenus par mise en oeuvre du procédé selon la revendication 20, **caractérisés en ce que** ces anticorps ont une capacité protectrice vis-à-vis de l'infection d'un hôte humain ou animal par des Bordetella de type *B. pertussis, B. parapertussis* et/ou *B. bronchiseptica.*

24. Anticorps monoclonaux produits par l'hybridome B5-4 déposé à la CNCM sous le n° I-1734 ou par l'hybridome E17-21 déposé à la CNCM sous le n°I-1733.

25. Anticorps monoclonaux dirigés contre une séquence d'acides aminés selon l'une quelconque des revendications 3 à 5.

26. Composition pharmaceutique **caractérisée en ce qu'**elle comprend à titre de principe actif des anticorps monoclonaux selon l'une quelconque des revendications 21 à 25.

27. Anticorps monoclonaux selon l'une quelconque des revendications 21 à 25, pour l'utilisation comme médicament en immunothérapie chez un hôte infecté par *B. pertussis* et/ou *B. parapertussis* et/ou *B. bronchiseptica.*

28. Hybridome **caractérisé en ce qu'**il s'agit de l'hybridome B5-4 déposé à la CNCM sous le n° I-1734 ou de l'hybridome E17-21 déposé à la CNCM sous le n°I-1733.

29. Procédé de détection *in vitro* d'une infection par une Bordetella notamment par *B. pertussis, B. parapertussis* ou *B. bronchiseptica*, **caractérisé en ce que** l'on met en contact un échantillon de fluide biologique d'un patient ou d'un animal susceptible d'être infecté par Bordetella, avec des anticorps monoclonaux selon l'une quelconque des revendications 21 à 25 et **en ce que** l'on détecte une réaction immunologique entre lesdits anticorps monoclonaux et les bactéries du genre Bordetella lorsqu'elles sont présentes dans l'échantillon.

30. Procédé de détection *in vitro* d'une infection par une Bordetella notamment par *B. pertussis, B. parapertussis* ou *B. bronchiseptica*, **caractérisé en ce que** l'on met en contact un échantillon de fluide biologique d'un patient ou d'un animal susceptible d'être infecté par Bordetella, avec une séquence d'acides aminés selon l'une quelconque des revendications 1 à 6 et **en ce que** l'on détecte une réaction immunologique entre lesdites séquences d'acides aminés et des anticorps présents dans l'échantillon testé.

31. Composition pharmaceutique **caractérisée en ce qu'**elle comprend à titre de principe actif, des séquences nucléotidiques selon la revendication 10.

**Claims**

1. An amino acid sequence derived from the polypeptide sequence of adenylcyclase-hemolysin (AC-Hly), **characterized in that** it is capable of inducing the formation of protecting antibodies against an infection by *B. pertussis* and/or *B. parapertussis* and/or *B. bronchiseptica* and **in that** it is selected from the following concatenations:

   a) the sequence of a truncated protein comprising the concatenation of amino acids located approximately between position 910, preferably 913, and the last C-terminal amino acid of the polypeptide sequence of the AC-Hly of *B. pertussis* or a sequence corresponding thereto in *B. parapertussis* or in *B. bronchiseptica,* said sequence comprising a modification by addition of a fatty acid between approximately amino acids 980 and approximately 985, preferably at amino acid 983;

b) a sequence having a three-dimensional conformation identical to or analogous with that of the corresponding polypeptide sequence of the AC-Hly of *B. pertussis, B. parapertussis* or *B. bronchiseptica*, comprising a concatenation of amino acids as defined above in a) and a concatenation of 16 to 500 amino acids comprising a sequence consisting of a concatenation of amino acids 385 to 400, 385 to 450 or 385 to 500 of the polypeptide sequence of the AC-Hly of *B. pertussis* or a sequence corresponding thereto in *B. parapertussis* or in *B. bronchiseptica*, said two concatenations being linked via an antigenic sequence derived from a protein which is different from AC-Hly;

c) a sequence containing a three dimensional conformation identical to or analogous with that of the corresponding polypeptide sequence of the AC-Hly of *B. pertussis, B. parapertussis* or *B. bronchiseptica,* comprising a concatenation of amino acids as defined above in a) and a concatenation of 16 to 500 amino acids comprising a sequence consisting of a concatenation of amino acids 385 to 400, 385 to 450, or 385 to 500 of the polypeptide sequence of the AC-Hly of *B. pertussis* or a sequence corresponding thereto in *B. parapertussis* or in *B. bronchiseptica*, said two concatenations being either contiguous, or linked via the concatenation of amino acids naturally present between the two concatenations defined above within the AC-Hly of *B. pertussis, B. parapertussis* or *B. bronchiseptica*, the amino acid sequence obtained being truncated compared with the AC-Hly sequence of Bordetella.

2. An amino acid sequence according to claim 1, **characterized in that** it is in the form of a polypeptide having a three dimensional conformation identical to or analogous with that of the corresponding polypeptide sequence of the AC-Hly of *B. pertussis* or *B. parapertussis* or *B. bronchiseptica,* **in that** it comprises the amino acid concatenation located between approximately position 900, in particular 910, and approximately the last C-terminal amino acid of the polypeptide sequence of the AC-Hly of *B. pertussis* or a sequence corresponding thereto in *B. parapertussis* or in *B. bronchiseptica,* said sequence further comprising a modification by addition of a fatty acid between approximately amino acid 980 and approximately amino acid 985, in particular at amino acid 983.

3. An amino acid sequence according to claim 1, **characterized in that** it is formed by the concatenation of amino acids included between the amino acid in approximately position 385 and approximately the last C-terminal amino acid of the polypeptide sequence of the AC-Hly of *B. pertussis* or a sequence corresponding thereto in *B. parapertussis* or in *B. bronchiseptica,* said sequence comprising a modification by addition of a fatty acid between approximately amino acid 980 and approximately amino acid 985, preferably at amino acid 983.

4. An amino acid sequence according to claim 1, **characterized in that** it is the polypeptide sequence of the AC-Hly modified by deletion of a polypeptide fragment termed ΔCla corresponding to the concatenation of amino acids 827 to 887 of the polypeptide sequence of the AC-Hly of *B. pertussis* or a sequence corresponding thereto in *B. parapertussis* or in *B. bronchiseptica*, the sequence obtained comprising a modification by addition of a fatty acid between approximately amino acid 980 and approximately amino acid 985, preferably at amino acid 983.

5. An amino acid sequence according to claim 1, **characterized in that** it is the polypeptide sequence of the AC-Hly modified by deletion of a polypeptide fragment termed ΔH corresponding to the concatenation of amino acids 385 to 827 of the polypeptide sequence of the AC-Hly of *B. pertussis* or a sequence corresponding thereto in *B. parapertussis* or in *B. bronchiseptica*, the sequence obtained comprising a modification by addition of a fatty acid between approximately amino acid 980 and approximately amino acid 985, preferably at amino acid 983.

6. An amino acid sequence according to any one of claims 1 to 5, **characterized in that** the modification by addition of a fatty acid is palmitoylation.

7. A polypeptide composition, **characterized in that** it comprises a sequence of *B. pertussis* according to any one of claims 1 to 6 and a sequence of *B. parapertussis* according to any one of claims 1 to 6.

8. A polypeptide composition according to claim 7, **characterized in that** it further comprises a sequence of *B. bronchiseptica* according to any one of claims 1 to 6.

9. A polypeptide composition, **characterized in that** it comprises a sequence of *B. parapertussis* according to any one of claims 1 to 6 and a sequence of *B. bronchiseptica* according to any one of claims 1 to 6.

10. A nucleotide sequence coding for an amino acid sequence according to any one of claims 1 to 5.

11. An immunogenic composition, **characterized in that** it comprises one or more sequences according to any one of claims 1 to 6.

12. An immunogenic composition according to claim 11, **characterized in that** it further comprises a bacterial extract containing the expression products of the vrg genes of a Bordetella strain selected from *B. pertussis, B. parapertussis* and *B. bronchiseptica* or a portion of said expression products, sufficient to induce an immune response in a host to which the extract would be administered.

13. An immunogenic composition according to claim 11, **characterized in that** it further comprises one or more adhesins or toxins of *B. pertussis, B. parapertussis* or *B. bronchiseptica* selected from FHA, AGGs or PRN and PTX or a portion of said proteins, sufficient to induce an immune response in a host to which the extract would be administered.

14. An immunogenic composition according to any one of claims 11 to 13, **characterized in that** the strain of *B. pertussis* from which the amino acid sequences derived from the AC-Hly toxin are obtained and if appropriate the proteins expressed by the vrg genes is the HAV strain deposited with the CNCM with accession number I-1485.

15. An immunogenic composition according to any one of claims 11 to 13, **characterized in that** the strain of *B. parapertussis* from which the amino acid sequences derived from the AC-Hly toxin and the proteins expressed by the vrg genes are obtained is strain number 1 deposited with the CNCM with accession number I-1498.

16. An immunogenic composition according to any one of claims 11 to 13, **characterized in that** the strain of *B. bronchiseptica* from which the amino acid sequences derived from the AC-Hly toxin and the proteins expressed by the vrg genes are obtained is strain 9.73 S deposited with the CNCM with accession n° I-858.

17. A vaccinating composition, **characterized in that** it comprises, as the active principle, an immunogenic composition according to any one of claims 11 to 16, in association with a pharmaceutically acceptable vehicle and, if appropriate, an adjuvant.

18. A method for preparing a polyclonal serum, comprising the steps of:

   ● immunizing a non-human animal with one or more amino acid sequences in accordance with any one of claims 1 to 5, associated or otherwise with an adjuvant;
   ● recovering the antibodies formed which are capable of recognizing the amino acid sequences used for immunization.

19. A preparation method according to claim 18, **characterized in that** animal immunization is carried out with one or more amino acid sequences according to any one of claims 1 to 6 and an immunogenic composition comprising antigens which are different from AC-Hly.

20. A method for preparing monoclonal antibodies recognizing the AC-Hly of *B. pertussis* and/or *B. parapertussis* and/or *B. bronchiseptica*, comprising the steps of:

   ● immunizing a non-human animal, for example a Balb/c mouse, with an amino acid sequence according to any one of claims 1 to 5;
   ● fusing spleen cells of the immunized animal with myeloma cells to form a hybridoma;
   ● culturing the hybridoma under conditions which allow the production of antibody;
   ● recovering antibodies directed against the amino acid sequence used for immunization.

21. Monoclonal antibodies, **characterized in that** they are directed against the sequence of amino acids 385 to 400 of the AC-Hly of *B. pertussis.*

22. Monoclonal antibodies, **characterized in that** they are specifically directed against the sequence of the last 217 amino acids of the AC-Hly of *B. pertussis,* in particular the sequence of amino acids 1488 to 1705 of the AC-Hly of *B. pertussis* or the sequence of amino acids 1489 to 1706 of the AC-Hly of *B. pertussis.*

23. Monoclonal antibodies according to claim 21 or 22 obtained by carrying out the method of claim 20, **characterized in that** said antibodies have a protective capacity against infection of a human or animal host by Bordetella of the *B. pertussis, B. parapertussis* and/or *B. bronchiseptica* type.

24. Monoclonal antibodies produced by the B5-4 hybridoma deposited with the CNCM with accession number I-1734 or by the E17-21 hybridoma deposited with the CNCM with accession number I-1733.

25. Monoclonal antibodies directed against an amino acid sequence in accordance with any one of claims 3 to 5.

26. A pharmaceutical composition, **characterized in that** it comprises, as an active principle, monoclonal antibodies according to any one of claims 21 to 25.

27. Monoclonal antibodies according to any one of claims 21 to 25, for use as a drug in immunotherapy in a host infected with *B. pertussis* and/or *B. parapertussis* and/or *B. bronchiseptica.*

28. A hybridoma, **characterized in that** it is the B5-4 hybridoma deposited with the CNCM with accession number I-1734 or the hybridoma E17-21 deposited with the CNCM with accession number I-1733.

29. A method for the *in vitro* detection of an infection by a Bordetella, in particular by *B. pertussis, B. parapertussis* or *B. bronchiseptica,* **characterized in that** a sample of biological fluid from a patient or an animal suspected of being infected by Bordetella is brought into contact with monoclonal antibodies according to any one of claims 21 to 25, and **in that** an immunological reaction between said monoclonal antibodies and bacteria of the genus Bordetella is detected when said bacteria are present in the sample.

30. A method for the *in vitro* detection of an infection by a Bordetella, in particular by *B. pertussis, B. parapertussis* or *B. bronchiseptica,* **characterized in that** a sample of biological fluid from a patient or an animal suspected of being infected by Bordetella is brought into contact with an amino acid sequence in accordance with any one of claims 1 to 6, and **in that** an immunological reaction between said amino acid sequences and antibodies present in the test sample is detected.

31. A pharmaceutical composition, **characterized in that** it comprises, as an active principle, nucleotide sequences according to claim 10.

**Patentansprüche**

1. Aminosäuresequenz, von der Polypeptidsequenz von Adenylate-Zyklase-Hämolysin (AC-Hly) stammend, **dadurch gekennzeichnet, dass** sie in der Lage ist, die Bildung schützender Antikörper gegen eine Infektion durch *B. pertussis* und/oder *B. parapertussis* und/oder *B. bronchiseptica* zu bewirken, und **dadurch**, dass sie aus den folgenden Ketten gewählt ist:

 a) der Sequenz eines gekürzten Proteins, die die Kette von Aminosäuren umfasst, die ungefähr zwischen der Position 910, vorzugsweise 913, und der letzten C-terminalen Aminosäure der AC-Hly-Polypeptidsequenz von *B. pertussis* oder einer der letztgenannten entsprechenden Sequenz bei *B. parapertussis* oder bei *B. bronchiseptica* angeordnet ist, wobei die Sequenz eine Modifikation durch Addition einer Fettsäure zwischen den Aminosäuren ungefähr 980 und ungefähr 985 aufweist, vorzugsweise auf Höhe der Aminosäure 983;
 b) einer Sequenz mit einer dreidimensionalen Struktur, die zu jener der entsprechenden AC-Hly-Polypeptidsequenz von *B. pertussis, B. parapertussis* oder *B. bronchiseptica* identisch oder analog ist, und die eine Aminosäurenkette wie unter a) definiert aufweist sowie eine Kette von 16 bis 500 Aminosäuren, die eine Sequenz aufweist, die aus der Kette von Aminosäuren 385 bis 400, 385 bis 450 oder 385 bis 500 der AC-Hly-Polypeptidsequenz von *B. pertussis* oder einer der vorgenannten entsprechenden Sequenz bei *B. parapertussis* oder bei *B. bronchiseptica* besteht, wobei die beiden Ketten mittels einer Antigen-Sequenz verbunden werden, die von einem Protein unterschiedlich zu AC-Hly stammt;
 c) einer Sequenz mit einer dreidimensionalen Struktur, die zu jener der entsprechenden AC-Hly-Polypeptidsequenz von *B. pertussis, B. parapertussis* oder *B. bronchiseptica* identisch oder analog ist, und die eine Aminosäurenkette wie unter a) definiert aufweist sowie eine Kette von 16 bis 500 Aminosäuren, die eine Sequenz aufweist, die aus der Kette von Aminosäuren 385 bis 400, 385 bis 450 oder 385 bis 500 der AC-Hly-Polypeptidsequenz von *B. pertussis* oder einer der vorgenannten entsprechenden Sequenz bei *B. parapertussis* oder bei *B. bronchiseptica* besteht, wobei die beiden Ketten entweder aneinander stoßen oder mittels der Aminosäurenkette verbunden werden, die natürlicherweise zwischen den beiden oben definierten Ketten in AC-Hly von *B. pertussis, B. parapertussis* oder *B. bronchiseptica* vorhanden ist, wobei die erhaltene Aminosäurensequenz bezogen auf die Sequenz von AC-Hly bei *Bordetella* verkürzt ist.

**2.** Aminosäurensequenz gemäß Anspruch 1, **dadurch gekennzeichnet, dass** sie die Form eines Polypeptids mit einer dreidimensionalen Struktur aufweist, die zu jener der entsprechenden AC-Hly-Polypeptidsequenz von *B. pertussis, B. parapertussis* oder *B. bronchiseptica* identisch oder analog ist, und **dadurch**, dass sie die Kette von Aminosäuren aufweist, die zwischen der Position ungefähr 900, insbesondere 910, und ungefähr der letzten C-terminalen Aminosäure der AC-Hly-Polypeptidsequenz von *B. pertussis* oder einer der vorgenannten entsprechenden Sequenz bei *B. parapertussis* oder bei *B. bronchiseptica* angeordnet ist, wobei die Sequenz des Weiteren eine Modifikation durch Addition einer Fettsäure zwischen den Aminosäuren ungefähr 980 und ungefähr 985 aufweist, vorzugsweise auf Höhe der Aminosäure 983.

**3.** Aminosäurensequenz gemäß Anspruch 1, **dadurch gekennzeichnet, dass** sie aus der Kette von Aminosäuren gebildet ist, die zwischen der Aminosäure an Position ungefähr 385 und ungefähr der letzten C-terminalen Aminosäure der AC-Hly-Polypeptidsequenz von *B. pertussis* oder einer der vorgenannten entsprechenden Sequenz bei *B. parapertussis* oder bei *B. bronchiseptica* angeordnet ist, wobei die Sequenz des Weiteren eine Modifikation durch Addition einer Fettsäure zwischen den Aminosäuren ungefähr 980 und ungefähr 985 aufweist, vorzugsweise auf Höhe der Aminosäure 983.

**4.** Aminosäurensequenz gemäß Anspruch 1, **dadurch gekennzeichnet, dass** es sich um die AC-Hly-Polypeptidsequenz handelt, die durch Deletion eines Polypeptidfragments modifiziert ist, das ΔCla genannt wird und der Kette von Aminosäuren 827 bis 887 der AC-Hly-Polypeptidsequenz von *B. pertussis* oder einer der vorgenannten entsprechenden Sequenz bei *B. parapertussis* oder bei *B. bronchiseptica* entspricht, wobei die erhaltene Sequenz eine Modifikation durch Addition einer Fettsäure zwischen den Aminosäuren ungefähr 980 und ungefähr 985 aufweist, vorzugsweise auf Höhe der Aminosäure 983.

**5.** Aminosäurensequenz gemäß Anspruch 1, **dadurch gekennzeichnet, dass** es sich um die AC-Hly-Polypeptidsequenz handelt, die durch Deletion eines Polypeptidfragments modifiziert ist, das ΔH genannt wird und der Kette von Aminosäuren 385 bis 827 der AC-Hly-Polypeptidsequenz von *B. pertussis* oder einer der vorgenannten entsprechenden Sequenz bei *B. parapertussis* oder bei *B. bronchiseptica* entspricht, wobei die erhaltene Sequenz eine Modifikation durch Addition einer Fettsäure zwischen den Aminosäuren ungefähr 980 und ungefähr 985 aufweist, vorzugsweise auf Höhe der Aminosäure 983.

**6.** Aminosäurensequenz gemäß einem der Ansprüche 1 bis 5, **dadurch gekennzeichnet, dass** die Modifikation durch Addition einer Fettsäure eine Palmitoylierung ist.

**7.** Polypeptidzusammensetzung, **dadurch gekennzeichnet, dass** sie eine Sequenz gemäß einem der Ansprüche 1 bis 6 von *B. pertussis* und eine Sequenz gemäß einem der Ansprüche 1 bis 6 von *B. parapertussis* aufweist.

**8.** Polypeptidzusammensetzung gemäß Anspruch 7, **dadurch gekennzeichnet, dass** sie des Weiteren eine Sequenz gemäß einem der Ansprüche 1 bis 6 von *B. bronchiseptica* aufweist.

**9.** Polypeptidzusammensetzung, **dadurch gekennzeichnet, dass** sie eine Sequenz gemäß einem der Ansprüche 1 bis 6 von *B. parapertussis* und eine Sequenz gemäß einem der Ansprüche 1 bis 6 von *B. bronchiseptica* aufweist.

**10.** Nukleotidsequenz, die für eine Aminosäurensequenz gemäß einem der Ansprüche 1 bis 5 kodiert.

**11.** Immunogene Zusammensetzung, **dadurch gekennzeichnet, dass** sie eine oder mehrere Sequenzen gemäß einem der Ansprüche 1 bis 6 aufweist.

**12.** Immunogene Zusammensetzung gemäß Anspruch 11, **dadurch gekennzeichnet, dass** sie des Weiteren ein bakterielles Extrakt aufweist, das die Expressionsprodukte der Gene vrg eines Bordetella-Stamms gewählt aus *B. pertussis, B. parapertussis* oder *B. bronchiseptica* oder einen Teil dieser Expressionsprodukte aufweist, der ausreichend ist, um eine Immunreaktion bei einem Wirt zu bewirken, dem das Extrakt verabreicht wurde.

**13.** Immunogene Zusammensetzung gemäß Anspruch 11, **dadurch gekennzeichnet, dass** sie des Weiteren eins oder mehrere Adhäsine oder Toxine von *B. pertussis, B. parapertussis* oder *B. bronchiseptica* aufweist, gewählt aus FHA, den AGGs oder PRN und PTX oder einem Teil dieser Proteine, der ausreichend ist, um eine Immunreaktion bei einem Wirt zu bewirken, dem das Extrakt verabreicht wurde.

**14.** Immunogene Zusammensetzung gemäß einem der Ansprüche 11 bis 13, **dadurch gekennzeichnet, dass** der Stamm von *B. pertussis,* aus dem die Aminosäurensequenzen erhalten werden, die von dem AC-Hly-Toxin und gegebenenfalls den Proteinen stammen, die von den Genen vrg exprimiert werden, der Stamm HAV ist, der beim CNCM unter der Nummer I-1485 angemeldet wurde.

**15.** Immunogene Zusammensetzung gemäß einem der Ansprüche 11 bis 13, **dadurch gekennzeichnet, dass** der Stamm von *B. parapertussis,* aus dem die Aminosäurensequenzen erhalten werden, die von dem AC-Hly-Toxin und den Proteinen stammen, die von den Genen vrg exprimiert werden, der Stamm Nr. 1 ist, der beim CNCM unter der Nummer I-1498 angemeldet wurde.

**16.** Immunogene Zusammensetzung gemäß einem der Ansprüche 11 bis 13, **dadurch gekennzeichnet, dass** der Stamm von *B. bronchiseptica,* aus dem die Aminosäurensequenzen erhalten werden, die von dem AC-Hly-Toxin und den Proteinen stammen, die von den Genen vrg exprimiert werden, der Stamm 9.73 S ist, der beim CNCM unter der Nummer I-858 angemeldet wurde.

**17.** Impfzusammensetzung, **dadurch gekennzeichnet, dass** sie als Wirkstoff eine immunogene Zusammensetzung gemäß einem der Ansprüche 11 bis 16 in Verbindung mit einem pharmazeutisch akzeptablen Träger und gegebenenfalls einem Adjuvans aufweist.

**18.** Verfahren zur Herstellung eines polyklonalen Serums, das die folgenden Schritte aufweist:

- Immunisierung eines nicht humanen Tieres mit einer oder mehreren Aminosäurensequenzen gemäß einem der Ansprüche 1 bis 5 in Verbindung mit einem Adjuvans oder nicht,
- Gewinnung der gebildeten Antikörper, die in der Lage sind, die für die Immunisierung verwendeten Aminosäurensequenzen zu erkennen.

**19.** Herstellungsverfahren gemäß Anspruch 18, **dadurch gekennzeichnet, dass** die Immunisierung des Tiers mit einer oder mehreren Aminosäurensequenzen gemäß einem der Ansprüche 1 bis 6 und eine immunogenen Zusammensetzung, die die unterschiedlichen Antikörper von AC-Hly aufweist, durchgeführt wird.

**20.** Verfahren zur Herstellung monoklonaler Antikörper, die AC-Hly von *B. pertussis* und/oder *B. parapertussis* und/oder *B. bronchiseptica* erkennen, welches die folgenden Schritte aufweist:

- Immunisierung eines nicht humanen Tieres, beispielsweise einer Maus Balb/c, mit einer Aminosäurensequenz gemäß einem der Ansprüche 1 bis 5,
- Fusion der Milzzellen des immunisierten Tieres mit Myelomzellen, um ein Hybridom zu bilden;
- Kultur des Hybridoms unter Konditionen, die das Erzeugen von Antikörpern ermöglichen;
- Gewinnung der Antikörper, die sich gegen die für die Immunisierung verwendete Aminosäurensequenz richtet.

**21.** Monoklonale Antikörper, **dadurch gekennzeichnet, dass** sie sich gegen die Sequenz von Aminosäuren 385 bis 400 von AC-Hly von *B. pertussis* richten.

**22.** Monoklonale Antikörper, **dadurch gekennzeichnet, dass** sie sich spezifisch gegen die Sequenz der 217 letzten Aminosäuren von AC-Hly von *B. pertussis,* insbesondere die Sequenz von Aminosäuren 1488 bis 1705 von AC-Hly von *B. pertussis* oder die Sequenz von Aminosäuren 1489 bis 1706 von AC-Hly von *B. bronchiseptica,* richten.

**23.** Monoklonale Antikörper gemäß Anspruch 21 oder 22, wie durch das Verfahren gemäß Anspruch 20 erhalten, **dadurch gekennzeichnet, dass** die Antikörper eine Schutzfähigkeit gegen die Infektion eines menschlichen oder tierischen Wirts durch Bordetella vom Typ *B. pertussis, B. parapertussis* oder *B. bronchiseptica* aufweisen.

**24.** Monoklonale Antikörper, erzeugt durch das Hybridom B5-4, angemeldet beim CNCM unter der Nr. I-1734, oder das Hybridom E17-21, angemeldet bei CNCM unter der Nr. I-1733.

**25.** Monoklonale Antikörper, die sich gegen eine Aminosäurensequenz gemäß einem der Ansprüche 3 bis 5 richten.

**26.** Pharmazeutische Zusammensetzung, **dadurch gekennzeichnet, dass** sie als Wirkstoff monoklonale Antikörper gemäß einem der Ansprüche 21 bis 25 aufweist.

**27.** Monoklonale Antikörper gemäß einem der Ansprüche 21 bis 25 zur Verwendung als Medikament bei der Immuntherapie bei einem durch *B. pertussis* und/oder *B. parapertussis* und/oder *B. bronchiseptica* infizierten Wirt.

28. Hybridom, **dadurch gekennzeichnet, dass** es sich um das Hybridom B5-4, angemeldet beim CNCM unter der Nr. 1-1734, oder das Hybridom E17-21, angemeldet beim CNCM unter der Nr. I-1733, handelt.

29. Verfahren zur *in vitro*-Detektion einer Infektion durch eine Bordetella, insbesondere durch *B. pertussis, B. parapertussis* oder *B. bronchiseptica,* **dadurch gekennzeichnet, dass** eine Probe einer biologischen Flüssigkeit eines Patienten oder eines Tiers, geeignet, mit Bordetella infiziert zu sein, mit den monoklonalen Antikörpern gemäß einem der Ansprüche 21 bis 25 in Kontakt gebracht wird, und **dadurch**, dass eine Immunreaktion zwischen den monoklonalen Antikörpern und den Bakterien der Gattung Bordetella, sofern diese in der Probe vorhanden sind, detektiert wird.

30. Verfahren zur in vitro-Detektion einer Infektion durch eine Bordetella, insbesondere durch *B. pertussis, B. parapertussis* oder *B. bronchiseptica,* **dadurch gekennzeichnet, dass** eine Probe einer biologischen Flüssigkeit eines Patienten oder eines Tiers, geeignet, mit Bordetella infiziert zu sein, mit einer Aminosäurensequenz gemäß einem der Ansprüche 1 bis 6 in Kontakt gebracht wird, und **dadurch**, dass eine Immunreaktion zwischen den Aminosäurensequenzen und den in der getesteten Probe vorhandenen Antikörpern detektiert wird.

31. Pharmazeutische Zusammensetzung, **dadurch gekennzeichnet, dass** sie als Wirkstoff die Nukleotidsequenzen gemäß Anspruch 10 aufweist.

EP 0 787 796 B1

FIGURE 1

FIGURE 2

FIGURE 3A

A

FIGURE 3B

B

FIGURE 3C

C

FIGURE 4

FIGURE 5(A)

Bordetella pertussis adenylate cyclase

# FIGURE 5(B)

EP 0 787 796 B1

FIGURE 5(C)

EP 0 787 796 B1

Séquence AC-Hly B. bronchiseptica

```
atg cag caa tcg cat cag gct ggt tac gca aac gcc gcc gac cgg gag tct ggc atc ccc
 M   Q   Q   S   H   Q   A   G   Y   A   N   A   A   D   R   E   S   G   I   P

gca gcc gta ctc gat ggc atc aag gcc gtg gcg aag gaa aaa aac gcc aca ttg atg ttc
 A   A   V   L   D   G   I   K   A   V   A   K   E   K   N   A   T   L   M   F

cgc ctg gtc aac ccc cat tcc acc agc ctg att gcc gaa ggg gtg gcc acc aaa gga ttg
 R   L   V   N   P   H   S   T   S   L   I   A   E   G   V   A   T   K   G   L

ggc gtg cac gcc aag tcg tcc gat tgg ggg ttg cag gcg ggc tac att ccc gtc aac ccg
 G   V   H   A   K   S   S   D   W   G   L   Q   A   G   Y   I   P   V   N   P

aat ctt tcc aaa ctg ttc ggc cgt gcg ccc gag gtg atc gcg cgg gcc gac aac gac gtc
 N   L   S   K   L   F   G   R   A   P   E   V   I   A   R   A   D   N   D   V

aac agc agc ctg gcg cat ggc cat acc gcg gtc gac ctg acg ctg tcg aaa gag cgg ctt
 N   S   S   L   A   H   G   H   T   A   V   D   L   T   L   S   K   E   R   L

gac tat ctg cgg caa gcg ggc ctg gtc acc ggc atg gcc gat ggc gtg gtc gcg agc aac
 D   Y   L   R   Q   A   G   L   V   T   G   H   A   D   G   V   V   A   S   N

cac gca ggc tac gag cag ttc gag ttt cgc gtg aag gaa acc tcg gac ggg cgc tat gcc
 H   A   G   Y   E   Q   F   E   F   R   V   K   E   T   S   D   G   R   Y   A

gtg cag tat cgc cgc aag ggc ggc gac gat ttc gag gcg gtc aag gtg atc ggc aat gcc
 V   Q   Y   R   R   K   G   G   D   D   F   E   A   V   K   V   I   G   N   A

gcc ggt att cca ctg acg gcg gat atc gac atg ttc gcc atc atg ccg cat ctg tcc aac
 A   G   I   P   L   T   A   D   I   D   M   F   A   I   H   P   H   L   S   N

ttc cgc gac tcg gcg cgc agt tcg gtg acc agc ggc gat tcg gtg acc gat tac ctg gcg
 F   R   D   S   A   R   S   S   V   T   S   G   D   S   V   T   D   Y   L   A

cgc acg cgg cgg gcc gcc agc gag gcc acg ggc ggc ctg gat cgc gaa cgc atc gac ttg
 R   T   R   R   A   A   S   E   A   T   G   G   L   D   R   E   R   I   D   L

ttg tgg aaa atc gct cgc gcc ggc gcc cgt tcc gca gtg ggc acc gag gcg cgt cgc cag
 L   W   K   I   A   R   A   G   A   R   S   A   V   G   T   E   A   R   R   Q

ttc cgc tac gac ggc gac atg aat atc ggc gtg atc acc gat ttc gag ctg gaa gtg cgc
 F   R   Y   D   G   D   M   N   I   G   V   I   T   D   F   E   L   E   V   R
                                             A

aat gcg ctg aac agg cgg gcg cac gcg gtc ggc agg cag gac gtg gtc cag cat ggc act
 N   A   L   N   R   R   A   H   A   V   G   R   Q   D   V   V   Q   H   G   T

gag cag aac aat cct ttc ccg gag gca gat gag aag att ttc gtc gta tcg gcc acc ggt
 E   Q   N   N   P   F   P   E   A   D   E   K   I   F   V   V   S   A   T   G

gaa agc cag atg ctc acg cgc ggg caa ctg aag gaa tac att ggc cag cag cgc ggc gag
 E   S   Q   M   L   T   R   G   Q   L   K   E   Y   I   G   Q   Q   R   G   E

ggc tat gtc ttc tac gag aac cgt gcg tac ggc gtg gcg ggg aaa agc ctg ttc gac gat
 G   Y   V   F   Y   E   N   R   A   Y   G   V   A   G   K   S   L   F   D   D
                             S   G                   F

ggg ctg gga gcc gcg ccc ggc gtg ccg ggg cga cgt tcg aag tcc tcg ccg gat gta ctg
 G   L   G   A   A   P   G   V   P   G   R   R   S   K   S   S   P   D   V   L

gaa acg gtg ccg gcg tca ccc gga ttg cgg cgg ccg tcg ctg ggc gca gtg gaa cgc cag
 E   T   V   P   A   S   P   G   L   R   R   P   S   L   G   A   V   E   R   Q

gat tcc ggc tat gac agc ctt gat ggg gtg gga tcg cga tcg ttc tcg ttg ggc gag gtg
 D   S   G   Y   D   S   L   D   G   V   G   S   R   S   F   S   L   G   E   V

tcc gac atg gcc gcc gtg gaa gcg gcg gaa ctg gaa atg acc cgg caa gtc ttg cac gcc
 S   D   M   A   A   V   E   A   A   E   L   E   M   T   R   Q   V   L   H   A

ggg gcg cgg cag gac gat gcc gag ccg ggc gtg agc ggt gcg tcg gcg cac tgg ggg cag
 G   A   R   Q   D   D   A   E   P   G   V   S   G   A   S   A   H   W   G   Q

gg gcg ctg caa ggc gcc cag gcg gtg gcg gcg gcg cag cgg ctg gtt cat gcc att gcc
    A   L   Q   G   A   Q   A   V   A   A   A   Q   R   L   V   H   A   I   A
```

FIGURE 6(A)

33

```
1441/481
ctg atg acg caa ttc ggc cgg gcc ggt tcc acc aac acg ccg cag gaa gcg gcc tcg ttg
 L   M   T   Q   F   G   R   A   G   S   T   N   T   P   Q   E   A   A   S   L
1501/501
tcg gcg gcc gtg ttc ggc ttg ggc gag gcc agc agc gcc gtg gcc gaa acc gtg agc ggt
 S   A   A   V   F   G   L   G   E   A   S   S   A   V   A   E   T   V   S   G
1561/521
ttt ttc cgc ggg tct tcg cgc tgg gcc ggc ggt ttc ggc gtg gct ggc ggc gcg atg gcg
 F   F   R   G   S   S   R   W   A   G   G   F   G   V   A   G   G   A   H   A
                        A A
1621/541
ctg gga ggc ggc atc ggc gcc gtt ggc gcc ggg atg tcg ttg acc gat gac gcg ccg gcc
 L   G   G   G   I   G   A   V   G   A   G   M   S   L   T   D   D   A   P   A
1681/561
gga cag aag gcc gcc gcc ggc gcc gag atc gcg ctg cag ttg aca ggt gga acg gtc gag
 G   Q   K   A   A   A   G   A   E   I   A   L   Q   L   T   G   T   V   E
1741/581
ctg gct tct tcc atc gcg ttg gcg ctg gcc gcg gcg cgc ggc gtg acc agc ggc ttg cag
 L   A   S   S   I   A   L   A   L   A   A   A   R   G   V   T   S   G   L   Q
1801/601
gtg gcg ggg gcg tcg gcc ggg gcg gct gcc ggc gca ttg gcc gcg gcg ctc agt ccc atg
 V   A   G   A   S   A   G   A   A   A   G   A   L   A   A   A   L   S   P   M
1861/621
gag atc tac ggc ctg gtg cag caa tcg cac tat gcg gat cag ctg gac aag ctg gcg cag
 E   I   Y   G   L   V   Q   Q   S   H   Y   A   D   Q   L   D   K   L   A   Q
1921/641
gaa tcg agc gca tac ggt tac gag ggc gac gcc ttg ctg gcc cag ctg tat cgc gac aag
 E   S   S   A   Y   G   Y   E   G   D   A   L   L   A   Q   L   Y   R   D   K
1981/661
acg gcc gcc gag ggc gcc gtc gcc ggc gtc tcc gcc gtc ctg agc acg gtg ggg gct gcg
 T   A   A   E   G   A   V   A   G   V   S   A   V   L   S   T   V   G   A   A
2041/681
gtg tcg atc gcc gcg gcg gcc agc gtg gta ggc gcc ccg gtg gcg gtg gtc act tcc ttg
 V   S   I   A   A   A   A   S   V   V   G   A   P   V   A   V   V   T   S   L
2101/701
ttg acc ggg gct ctc aac ggc atc ctg cgc ggc gtg cag cag ccc atc atc gaa aag ctg
 L   T   G   A   L   N   G   I   L   R   G   V   Q   Q   P   I   I   E   K   L
2161/721
gcc aat gat tac gct cgc aag atc gac gag ctg ggc ggg ccg caa gcg tac ttc gag aaa
 A   N   D   Y   A   R   K   I   D   E   L   G   G   P   Q   A   Y   F   E   K
2221/741
aac ctg cag gcg cgt cac gaa caa ctg gcc aat tcg gac ggc cta cgg aaa atg ctg gcc
 N   L   Q   A   R   H   E   Q   L   A   N   S   D   G   L   R   K   M   L   A
2281/761
gac ctg cag gcc ggg tgg aac gcc agc agc gtg atc ggg gtg cag acg aca gag att tcc
 D   L   Q   A   G   W   N   A   S   S   V   I   G   V   Q   T   T   E   I   S
                                                                            V
2341/781
aag tcg gcg ctc gaa ctg gcc gcc att acc ggc aac gcg gac aac ctg aaa tcc gcc gac
 K   S   A   L   E   L   A   A   I   T   G   N   A   D   N   L   K   S   A   D
                     V
2401/801
gtg ttc gtg gac cgc ttc atc cag ggc gag cgg gtg gcc ggc cag ccg gtg gta ctc gac
 V   F   V   D   R   F   I   Q   G   E   R   V   A   G   Q   P   V   V   L   D
2461/821
gtc gcc gcc ggc ggc atc gat atc gcc agc cgc aag ggc gag cgg ccg gcg ctg acg ttc
 V   A   A   G   G   I   D   I   A   S   R   K   G   E   R   P   A   L   T   F
2521/841
atc acg ccg ctg gcc gcg cca gga gaa gag cag cgc cgg cgc acg aaa acg ggc aag agc
 I   T   P   L   A   A   P   G   E   E   Q   R   R   R   T   K   T   G   K   S
2581/861
gaa ttc acc aca ttc gtc gag atc gtg ggc aag cag gac cgc tgg cgc atc cgg gac ggc
 E   F   T   T   F   V   E   I   V   G   K   Q   D   R   W   R   I   R   D   G
2641/881
gcg gcc gac acc acc atc gat ctg gcc aag gtg gtg tcg caa ctg gtc gac gcc aat ggc
 A   A   D   T   T   I   D   L   A   K   V   V   S   Q   L   V   D   A   N   G
                                 D
2701/901
gtg ctc aag cac agc atc aaa ctg gag gtg atc ggc gga gat ggc gat gat gtc gtg ctt
 V   L   K   H   S   I   K   L   E   V   I   G   G   D   G   D   D   V   V   L
2761/921
gcc aat gct tcg cgc atc cat tac gac ggc ggc gcg gga acc aac acg gtc agc tat gcc
 A   N   A   S   R   I   H   Y   D   G   G   A   G   T   N   T   V   S   Y   A
2821/941
gcc ctg ggc cga cag gat tcc att acc gtg tcc gcc gac ggg gaa cgt ttc aac gtg cgc
 A   L   G   R   Q   D   S   I   T   V   S   A   D   G   E   R   F   N   V   R
```

FIGURE 6(B)

```
881/961                                                    T
aag cag ttg aac aac gcc aac gtg tat cgc gaa ggc gtg gct acc cag aaa acc gcc tac
K   Q   L   N   N   A   N   V   Y   R   E   G   V   A   T   Q   K   T   A   Y
2941/981                                                                    V
ggc aag cgc acg gag aat gtc caa tac cgc cat gtc gag ctg gcc cgt gtc ggg caa ctg
G   K   R   T   E   N   V   Q   Y   R   H   V   E   L   A   R   V   G   Q   L
3001/1001
gtg gag gtc gac acg ctc gag cat gtg cag cac atc atc ggc ggg gcc ggc aac gat tcg
V   E   V   D   T   L   E   H   V   Q   H   I   I   G   G   A   G   N   D   S
3061/1021                                                  S
atc acc ggc aat gcg cac gac aac ttc ctg gcc ggc ggg gcg ggc gac gac agg ctg gat
I   T   G   N   A   H   D   N   F   L   A   G   G   A   G   D   D   R   L   D
                                                           Q               I
3121/1041
ggc ggc gcc ggc aac gac aca ctg gtc ggc ggc gag ggc cac aac acg gtc gtc ggc ggc
G   G   A   G   N   D   T   L   V   G   G   E   G   H   N   T   V   V   G   G
3181/1061
gct ggc gac gac gta ttc ctg cag gac ctg ggg gta tgg agc aac cag ctc gat ggc ggc
A   G   D   D   V   F   L   Q   D   L   G   V   W   S   N   Q   L   D   G   G
3241/1081
gcg ggc gtc gat acc gtg aag tac aac gtg cac cag cct tcc gag gaa cgc ctc gaa cgc
A   G   V   D   T   V   K   Y   N   V   H   Q   P   S   E   E   R   L   E   R
3301/1101
atg ggc gac acg ggc atc cat gcc gat ctt caa aag ggc acg gtc gag aag tgg ccg gcc
M   G   D   T   G   I   H   A   D   L   Q   K   G   T   V   E   K   W   P   A
3361/1121                                                                  R
ctg aac ctg ttc agc gtc gac cat gtc aag aat atc gag aat ctg cac ggc tcc agc ctg
L   N   L   F   S   V   D   H   V   K   N   I   E   N   L   H   G   S   S   L
3421/1141 R                           Q                           H
aac gac agc atc gcc ggc gac gac cgg gac aac gag ctc tgg ggc gac gat ggc aac gac
N   D   S   I   A   G   D   D   R   D   N   E   L   W   G   D   D   G   N   D
3481/1161 R
acg ata cac ggc cgg ggc ggc gac gat atc ctg cgc ggc ggc ctg ggc ctg gac acg ctg
T   I   H   G   R   G   G   D   D   I   L   R   G   G   L   G   L   D   T   L
3541/1181
tat ggc gag gac ggc aac gac atc ttc ctg cag gac gac gag acc gtc agc gat gac atc
Y   G   E   D   G   N   D   I   F   L   Q   D   D   E   T   V   S   D   D   I
3601/1201                                                  P               R
gac ggt ggc gcg gga ctg gac acc gtc gac tat tcc gcc atg atc cat gca ggc aag atc
D   G   G   A   G   L   D   T   V   D   Y   S   A   M   I   H   A   G   K   I
3661/1221                                                  R   E
gtt gcg ccg cat gaa tac ggc ttc ggg atc gag gcg gac ctg tcc gaa ggg tgg gtg cgc
V   A   P   H   E   Y   G   F   G   I   E   A   D   L   S   E   G   W   V   R
3721/1241 S   A   L       V               N           X
aag gcg gcc cgg cgc ggc atg gac tac tac gac agt gtc cgc agt gtc gaa aac gtc atc
K   A   A   R   R   G   M   D   Y   Y   D   S   V   R   S   V   E   N   V   I
3781/1261
ggc acg agc atg aag gat gtg ctc atc ggc gac gcg caa gcc aat acc ctg atg ggc cag
G   T   S   M   K   D   V   L   I   G   D   A   Q   A   N   T   L   M   G   Q
3841/1281
ggc ggc gac gat acc gtg cgc ggc ggc gac ggc gat gat ctg ctg ttc ggc ggc gac ggc
G   G   D   D   T   V   R   G   G   D   G   D   D   L   L   F   G   G   D   G
3901/1301
aac gac atg ctg tat gga gac gcc ggc aac gac acc ctc tac gga ggg ctg ggc gac gat
N   D   M   L   Y   G   D   A   G   N   D   T   L   Y   G   L   G   D   D
3961/1321                                                          Q
acc ctt gaa ggc ggc gcg ggc aac gat tgg ttc ggc cag acg ccg gcg cgc gag cat gac
T   L   E   G   G   A   G   N   D   W   F   G   Q   T   P   A   R   E   H   D
4021/1341             D                                   T
gtg ctg cgc ggc ggg gct ggg gtg gat acc gtg gat tac agc cag gcg ggc gcg cat gcc
V   L   R   G   G   A   G   V   D   T   V   D   Y   S   Q   A   G   A   H   A
4081  I       A
ggc gtt gcc acg ggt cgc atc ggg ctg ggt att ctg gcg gac ctg ggc gcc ggc cgc gtc
G   V   A   T   G   R   I   G   L   G   I   L   A   D   L   G   A   G   R   V
4141/1381
gac aag ctg ggc gag gcc ggc agc agc gcc tac gat acg gtt tcc ggc atc gaa aat gtg
D   K   L   G   E   A   G   S   S   A   Y   D   T   V   S   G   I   E   N   V
4701/1401
gtg ggc acg gaa ctg gcc gac cgc atc acg ggc gat gcg cag gcc aac gta ctg cgc ggc
V   G   T   E   L   A   D   R   I   T   G   D   A   Q   A   N   V   L   R   G
4261/1421
gcg ggt ggc gcc gac gtg ctt gcg ggc ggc gag ggc gac gat gtg ctg ctg ggc ggc gac
A   G   G   A   D   V   L   A   G   G   E   G   D   D   V   L   L   G   G   D
```

FIGURE 6(C)

35

4321/1441
ggc gac gac cag ctg tcg ggc gac gcc gga cgc gac cgc ttg tac ggc gaa gcc ggt gac
G   D   D   Q   L   S   G   D   A   G   R   D   R   L   Y   G   E   A   G   D
                                                                              R

4381/1461
gac tgg ttc ttc cag gat gcc gcc aat gcc ggc aat ctg ctc gac ggt ggt gac ggc aac
D   W   F   F · Q   D   A   A   N   A   G   N   L   L   D   G   G   D   G   N

4441/1481
gat acc gtg gat ttc agc ggc ccg ggc cgg ggc ctc gac gcc ggc gca aag ggc gta ttc
D   T   V   D   F   S   G   P   G   R   G   L   D   A   G   A   K   G   V   F

4501/1501
ctg agc ctg ggc aag ggg ttc gcc agc ctg atg gac gaa ccc gaa acc agc aac gtg ttg
L   S   L   G   K   G   F   A   S   L   M   D   E   P   E   T   S   N   V   L

4561 N
                                                    A
cgc cat atc gag aac gcc gtg ggc agc gtg cgt gat gac gtg ctg atc ggc gac gca ggc
R   H   I   E   N   A   V   G   S   V   R   D   D   V   L   I   G   D   A   G
                                                              G   G   A   G

4621/1541
gcc aac gtc ctc aat ggc ctg gcg ggc aac gac gtg ctg tcg gcg gcg ccg gcg gac gat
A   N   V   L   N   G   L   A   G   N   D   V   L   S   A   A   P   A   D   D

4681/1561
gtg ctg ctg ggc gac gag ggc tcg gac ctg ctc agc ggc gat gcg ggc aac gac gat ctg
V   L   L   G   D   E   G   S   D   L   L   S   G   D   A   G   N   D   D   L
                                                    V

4741/1581
ttc ggc ggg cag ggc gat gat acc tat ctg ttc ggg gcc ggg tac gga cat gac acg atc
F   G   G   Q   G   D   D   T   Y   L   F   G   A   G   Y   G   H   D   T   I

4801/1601
tac gaa tcg ggc ggc ggc cat gac acc atc cgt atc aac gcg ggg gcg gac cag ctg tgg
Y   E   S   G   G   G   H   D   T   I   R   I   N   A   G   A   D   Q   L   W

4861/1621
ttt gcg cgc cag ggc aac gac ctg gag atc cgc att ctt ggc acc gac gat gca ctt acc
F   A   R   Q   G   N   D   L   E   I   R   I   L   G   T   D   D   A   L   T
                                                    I

4921/1641
gtg cac gac tgg tat cgc gac gcc gat cac cgg gtg gaa gcc atc cat gcc gcc aac cag
V   H   D   W   Y   R   D   A   D   H   R   V   E   A   I   H   A   A   N   Q
4981 V           Q

gcc ata gac ccg gcc ggc atc gaa aag ctg gtc gag gca atg gcg cag tac ccg gac ccc
A   I   D   P   A   G   I   E   K   L   V   E   A   M   A   Q   Y   P   D   P

5041/1681
ggc gcg gcg gcg gct gcc ccg ccg gcg gcg cgc gtg ccg gac acg ctg atg cag tcc ctg
G   A   A   A   A   P   P   A   A   R   V   P   D   T   L   M   Q   S   L

5101/1701
gct gtc aac tgg cgc tga
A   V   N   W   R   •

FIGURE 6 (D)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **GLASER P. et al.** *Molec. Microb.,* 1988, vol. 2, 19-20
- **COOTE J.** *FEMS Microbiol. Rev.,* 1992, vol. 88, 137-162
- **BETSOU F. et al.** *Infect. Immun.,* 1993, vol. 61, 3583-3589
- **GLASER et al.** *Molec. Microb.,* 1988, 2-1930
- **BARRY E.M. et al.** *Journal of Bacteriology,* Janvier 1991, 720-726
- **LACEY B.** *J. Hyg,* 1960, vol. 58, 57-93
- **ARICO B. et al.** *Proc. Natl. Acad. Sci USA,* 1989, vol. 86, 6671-6675
- **UHL M. A. ; MILLER J.** *PRoc. Natl. Acad. Sci USA,* 1994, vol. 91, 1163-1167
- **BEATTIE D.T. et al.** *J. of Bacteriology,* vol. 93, 159-527
- **BEATTIES D. et al.** *Infect. Imm,* 1992, vol. 60, 571-577
- **WALDMANN T.** *Science,* Juin 1991, vol. 252, 1657-1662
- **WINTER G. et al.** *Immunology Today,* 1993, vol. 14 (6), 243-246
- **CARTER ; MAI et al.** *Proc. Natl. Acad. Sci. USA,* 1992, vol. 89, 4285-4289
- **SINGER et al.** *Journal of Immunology,* 01 Avril 1993, vol. 150 (7), 2844-2857
- **DONNELLY et al.** *Nature Médecine,* 1995, vol. 1 (6), 583-587
- **SEBO P. et al.** *Gene,* 1991, vol. 104, 19-24
- **SEBO P. et al.** *Mol. Microb.,* 1993, vol. 9, 999-1009
- **BETSOU F. et al.** *Infect. Immun,* 1993, vol. 61, 3583-3589
- **STAINER D.W. ; SCHOLTE M.J.** *J. Gen. Microb.,* 1971, vol. 63, 211-220
- **BETSOU F. ; P. SEBO ; N. GUISO.** CyaC-mediated activation is important not only for toxic but also for protective activities of Bordetella pertussis adenylate cyclase-hemolysin. *Infect. Immun,* 1993, vol. 61, 3583-3589
- **IWAKI M. ; ULLMANN A. ; P. SEBO.** *Oligomerization of the adenylate cyclase toxin of Bordetella pertussis : Evidence from in vitro complementation of individually inactive mutants*

- **SEBO P. ; P. GLASER ; H. SAKAMOTO ; A. ULLMANN.** High Level synthesis of adenylate cyclase toxin of Bordetella pertussis in a reconstructed Escherichia coli system. *Gene,* 1991, vol. 104, 19-24
- **BETSOU F. ; P. SEBO ; N. GUISO.** *Infect. Immun,* 1993, vol. 61, 3583-3589
- **LADANT D. ; C. BREZIN ; I. CRENON ; J.M. ALONSO ; N. GUISO.** Bordetella pertussis adenylate cyclase: purification, characterization and radioimmunoassay. *J. Biol. Chem.,* 1987, vol. 261, 16264-16269
- **BELLALOU J. ; H. SAKAMOTO ; D. LADANT ; C. GEOFFROY ; A. ULLMANN.** Deletions affecting hemolytic and toxin activities of Bordetella pertussis adenylate cyclase infect. *Immun,* 1990, vol. 58, 3242-3247
- **BRADFORD.** A rapid and sensitive method for the quantification of micrograms of protein, utilising the principle of protein-dye binding. *Anal. Biochem.,* vol. 72, 248-257
- **PRADELLES P. ; GRASSI J. ; CHABARDES D. ; GUISO N.** *Analytical Chemistry,* 1989, vol. 61, 447-450
- **GUISO N. ; M. SZATANIK ; M. ROCANDOURT.** Protective activity of Bordetella adenylate cyclase against bacterial colonization. *Microb. Pathog.,* 1991, vol. 11, 423-431
- **GUISO N. ROCANCOURT ; M. SZATANIK ; J.M. ALONSO.** Bordetella adenylate cyclase is a virulence associated factor and a protective antigen. *Molec. Pathog.,* 1989, vol. 7, 373-380
- **BETSOU et al.** CyaC-mediated activation is important not only for toxic but also for protective activities of Bordetella pertussis adenylate cyclase-hemolysin. *Infect. Immun,* vol. 61, 3583-3589
- **BETSOU F., SEBO ; N. GUISO.** CyaC-mediated activation is important not only for toxic but also for protective activities of Bordetella pertussis adenylate cyclase-hemolysin. *Infect. Immun,* 1993, vol. 61, 3583-3589
- **ARCINIAGA J.L. ; E.L. HEWLETT ; F.D. JONHSON ; A. DEFOREST ; S.G.F. WASSILAK ; I.M. ONORATO ; C.R. MANCLARK ; D.L. BURNS.** *J. Infect. Dis.,* 1991, vol. 163, 135-142
- **GUISO N. ; E. GRIMPREL ; I. ANJAK ; P. BÉGUÉ.** *Eur. J. Clin. Microbiol. and Infect. Dis.*
- **KHELEF, N. ; H. SAKAMOTO ; N. GUISO.** *Microb. Pathog.,* 1992, vol. 12, 227-235

- **GUISO, N. ; M. ROCANCOURT ; M. SZATANIK ; J.M. ALONSO.** *Molec. Pathog.,* vol. 7, 373-380

- **GUISO, N. M. SZATANIK ; M. ROCANCOURT.** *Microb. Pathog.,* 1991, vol. 11, 423-431